(19) Europäisches Patentamt
European Patent Office
Office européen des brevets

(11) EP 3 735 407 B1

(12) # EUROPEAN PATENT SPECIFICATION

(45) Date of publication and mention of the grant of the patent:
**01.04.2026 Bulletin 2026/14**

(21) Application number: **19700065.6**

(22) Date of filing: **04.01.2019**

(51) International Patent Classification (IPC):
***C07D 215/44*** (2006.01) ***A61K 31/4706*** (2006.01)
***A61P 35/00*** (2006.01)

(52) Cooperative Patent Classification (CPC):
**C07D 215/44**

(86) International application number:
**PCT/EP2019/050159**

(87) International publication number:
**WO 2019/134969 (11.07.2019 Gazette 2019/28)**

(54) **SUBSTITUTED HALO-QUINOLINE DERIVATES FOR USE IN THE TREATMENT OF LYMPHOMAS AND LEUKEMIA**

SUBSTITUIERTE HALOGEN-CHINOLINDERIVATE ZUR VERWENDUNG BEI DER BEHANDLUNG VON LYMPHOMEN UND LEUKÄMIE

DÉRIVÉS SUBSTITUÉS D'HALO-QUINOLÉINE POUR LEUR UTILISATION DANS LE TRAITEMENT DES LYMPHOMES ET DE LA LEUCÉMIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.01.2018 EP 18305005**

(43) Date of publication of application:
**11.11.2020 Bulletin 2020/46**

(73) Proprietors:
- **INSTITUT NATIONAL DE LA SANTE ET DE LA RECHERCHE MEDICALE - INSERM**
  **75013 Paris (FR)**
- **UNIVERSITÉ CÔTE D'AZUR**
  **06108 Nice Cedex 2 (FR)**
- **Centre National de la Recherche Scientifique**
  **75016 Paris (FR)**
- **Centre Hospitalier Universitaire de Nice**
  **06000 Nice (FR)**

(72) Inventors:
- **PASSERON, Thierry**
  **06204 Nice (FR)**
- **BENHIDA, Rachid**
  **06108 Nice Cedex 2 (FR)**
- **DAO, Pascal**
  **06000 Nice (FR)**
- **DE DONATIS, Gian Marco**
  **06000 Nice (FR)**
- **MARTIN, Anthony**
  **06000 Nice (FR)**
- **VERHOEYEN, Els**
  **06204 Nice (FR)**

(74) Representative: **Icosa**
**83 avenue Denfert-Rochereau**
**75014 Paris (FR)**

(56) References cited:
**CN-A- 102 846 623**

- **DAKSHANAMURTHY ET AL: "In-silico fragment-based identification of novel angiogenesis inhibitors", BIOORGANIC & MEDICINAL CHEMISTRY LETTERS, ELSEVIER, AMSTERDAM NL, vol. 17, no. 16, 17 July 2007 (2007-07-17), pages 4551 - 4556, XP022156487, ISSN: 0960-894X, DOI: 10.1016/J.BMCL.2007.05.104**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 735 407 B1

- RAIMO SAARI ET AL: "Microwave-assisted synthesis of quinoline, isoquinoline, quinoxaline and quinazoline derivatives as CB2 receptor agonists", BIOORGANIC & MEDICINAL CHEMISTRY, ELSEVIER, AMSTERDAM, NL, vol. 19, no. 2, 15 January 2011 (2011-01-15), pages 939 - 950, XP002674004, ISSN: 0968-0896, [retrieved on 20101209], DOI: 10.1016/J.BMC.2010.11.059
- BEAUCHARD ET AL: "Synthesis of original thiazoloindolo[3,2-c]quinoline and novel 8-N-substituted-11H-indolo[3,2-c]quinoline derivatives from benzotriazoles. Part I", TETRAHEDRON, ELSEVIER SIENCE PUBLISHERS, AMSTERDAM, NL, vol. 62, no. 8, 20 February 2006 (2006-02-20), pages 1895 - 1903, XP005269265, ISSN: 0040-4020, DOI: 10.1016/J.TET.2005.09.153
- DHANABAL T ET AL: "Heteroatom directed photoannulation: synthesis of indoloquinoline alkaloids: cryptolepine, cryptotackieine, cryptosanguinolentine, and their methyl derivatives", TETRAHEDRON, ELSEVIER SIENCE PUBLISHERS, AMSTERDAM, NL, vol. 62, no. 26, 26 June 2006 (2006-06-26), pages 6258 - 6263, XP025002141, ISSN: 0040-4020, [retrieved on 20060626], DOI: 10.1016/J.TET.2006.04.050
- N'DA DAVID D. ET AL: "Synthesis, in vitro antiplasmodial and antiproliferative activities of a series of quinoline-ferrocene hybrids", MEDICINAL CHEMISTRY RESEARCH, vol. 23, no. 3, 28 August 2013 (2013-08-28), US, pages 1214 - 1224, XP093255214, ISSN: 1054-2523, Retrieved from the Internet <URL:http://link.springer.com/article/10.1007/s00044-013-0748-4/fulltext.html> DOI: 10.1007/s00044-013-0748-4
- LIU DAN ET AL: "Synthesis and Anti-Tumor Activities of 4-Anilinoquinoline Derivatives", MOLECULES, vol. 21, no. 1, 23 December 2015 (2015-12-23), CH, pages 21, XP093255215, ISSN: 1420-3049, DOI: 10.3390/molecules21010021
- HAILE PAMELA A. ET AL: "The Identification and Pharmacological Characterization of 6-( tert -Butylsulfonyl)- N -(5-fluoro-1 H -indazol-3-yl) quinolin-4-amine (GSK583), a Highly Potent and Selective Inhibitor of RIP2 Kinase", JOURNAL OF MEDICINAL CHEMISTRY, vol. 59, no. 10, 4 May 2016 (2016-05-04), US, pages 4867 - 4880, XP093246546, ISSN: 0022-2623, DOI: 10.1021/acs.jmedchem.6b00211
- DENNY W A: "The 4-anilinoquinazoline class of inhibitors of the erbB family of receptor tyrosine kinases", IL FARMACO, ELSEVIER FRANCE * EDITIONS SCIENTIFIQUES ET MEDICALES, FR, vol. 56, 1 January 2001 (2001-01-01), pages 51 - 56, XP003015967, ISSN: 0014-827X, DOI: 10.1016/S0014-827X(01)01026-6
- NGUYEN THUY ET AL: "Functionalized acridin-9-yl phenylamines protected neuronal HT22 cells from glutamate-induced cell death by reducing intracellular levels of free radical species", BIOORGANIC & MEDICINAL CHEMISTRY LETTERS, ELSEVIER, AMSTERDAM NL, vol. 24, no. 7, 15 February 2014 (2014-02-15), pages 1830 - 1838, XP028835149, ISSN: 0960-894X, DOI: 10.1016/J.BMCL.2014.02.006
- MCANALLY DANIELLE ET AL: "Repurposing antimalarial aminoquinolines and related compounds for treatment of retinal neovascularization", PLOS ONE, vol. 13, no. 9, 12 September 2018 (2018-09-12), US, pages e0202436, XP093255216, ISSN: 1932-6203, DOI: 10.1371/journal.pone.0202436
- SHINMEE MAH ET AL: "Identification of 4-Phenoxyquinoline Based Inhibitors for L1196M Mutant of Anaplastic Lymphoma Kinase by Structure-Based Design", JOURNAL OF MEDICINAL CHEMISTRY, vol. 60, no. 22, 22 November 2017 (2017-11-22), pages 9205 - 9221, XP055549029, ISSN: 0022-2623, DOI: 10.1021/acs.jmedchem.7b01039
- OTEVREL J ET AL: "Antimycobacterial and photosynthetic electron transport inhibiting activity of ring-substituted 4-arylamino-7-chloroquinolinium chlorides", vol. 18, no. 9, 2 September 2014 (2014-09-02), pages 10649 - 10670, XP002761306, ISSN: 1420-3049, Retrieved from the Internet <URL:www.mdpi.com/journal/molecules> DOI: 10.3390/MOLECULES180910648

## Description

### Field of the invention:

The invention relates to substituted halo-quinoline derivatives which are active for the treatment of lymphomas and leukemia.

### Background of the invention:

The instant invention provides NIK inhibitors that are useful for the treatment of lymphomas and leukemia. They decrease EZH2 at the transcriptional level and induce the production of an IFN-γ response by the treated cells. These NIK inhibitors reduce the size of subcutaneous tumors without showing any specific toxicity, and when combined with anti-PD1 treatment lead to a dramatic reduction in tumor size with complete regression in some cases. Those effects are associated with a marked increase in the numbers and activation of type M1 macrophages, dendritic cells, Natural killer cells and T-cells within the treated tumors.

CN102846623 discloses the use of chloroquine or a derivative thereof in combination with a glucocorticoid receptor ligand for the treatment of various diseases. In CN102846623, the chloroquine derivatives having a phenyl ring attached to the -NH- in position 4 of the quinoline scaffold, carry all an aminoalkyl substituent, *i.e.* a substituent -alkylene-N(...)(...) on this phenyl ring.

### Summary of the invention:

The invention relates to compounds of general formula (I):

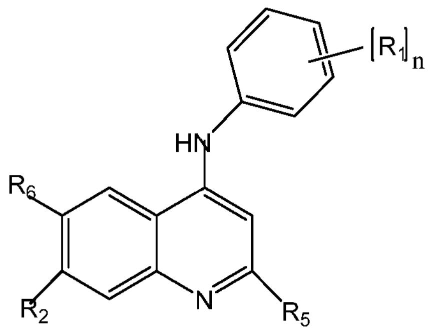

(I)

in which $R_1$, $R_2$, $R_5$, $R_6$ and n have the meanings indicated below, for use in the treatment of a disease selected from lymphomas and leukemia, and to pharmaceutical compositions for use in the treatment of a disease selected from lymphomas and leukemia, containing such compounds. In addition, these NIK inhibitors were shown to inhibit the non-canonical pathway and the development of lymphomas *in vitro* (B cell lymphoma results) and for inhibiting lymphoma development *in vivo.*

### Detailed description of the invention:

The invention relates more particularly to compounds of general formula (I):

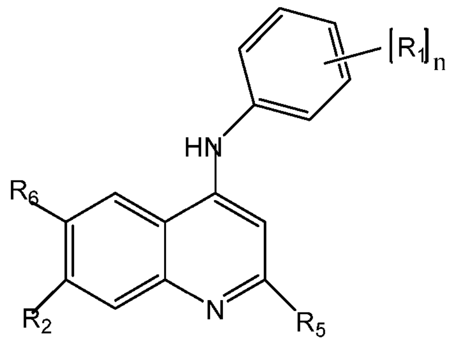

(I)

wherein

n is 2 and each $R_1$ is selected from alkyl, Halo, OH, O-alkyl, $NH_2$, NH-alkyl, N-(alkyl)$_2$, S-alkyl, $CF_3$, $OCF_3$, $OCF_2H$ and $O(CH_2)_{n1}O(CH_2)_{n2}CH_3$, $n_1$ being 1 to 4 and $n_2$ being 0 to 3, Halo being selected from Cl, F, Br and I;
or n is 1 and $R_1$ is selected from alkyl containing 2 to 6 carbon atoms, I, O-alkyl containing 2 to 6 carbon atoms, $NH_2$, NH-alkyl, N-(alkyl)$_2$, S-alkyl, $OCF_3$, $OCF_2H$ and $O(CH_2)_{n1}O(CH_2)_{n2}CH_3$, $n_1$ being 1 to 4 and $n_2$ being 0 to 3;
when n is 2, then it is also possible for $R_1$ to form with the phenyl group at the meta and para positions a dioxolane group or a 1,4 dioxane group,
$R_2$ is selected from H, Halo and $NR_3R_4$, Halo being defined as previously,
$R_3$ is H and $R_4$ is phenyl optionally substituted with one or more substituents selected from OH, O-alkyl and Halo, or $R_3$ and $R_4$ together form a $(CH_2)_2O(CH_2)_2$ chain, that is to say that $NR_3R_4$ forms a morpholino group,
$R_5$ is selected from H, cyclic radical, O-alkyl, O-$(CH_2)_{n1}$-(cyclic radical), and $(CH_2)_{n1}$-(cyclic radical), $n_1$ being defined as previously, said cyclic radical being chosen from

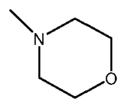 and 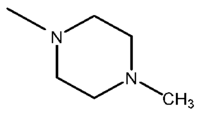,

$R_6$ is selected from H and Halo,
or a pharmaceutically acceptable salt and/or optical isomer, tautomer, or solvate thereof, for use in the treatment of a disease selected from lymphomas and leukemia.

The invention also relates to compounds of general formula (I):

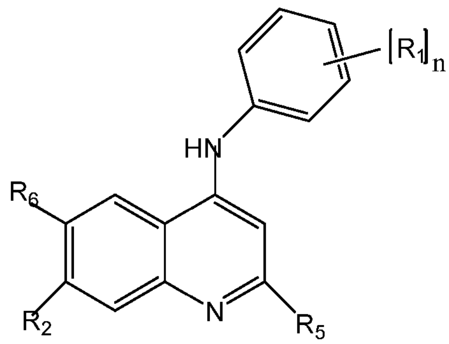

(I)

wherein

$R_1$ is selected from alkyl, Halo, OH, O-alkyl, $NH_2$, NH-alkyl, N-(alkyl)$_2$, S-alkyl, $CF_3$, $OCF_3$, $OCF_2H$ and $O(CH_2)_{n1}O(CH_2)_{n2}CH_3$, $n_1$ being 1 to 4 and $n_2$ being O to 3, Halo being selected from Cl, F, Br and I;
n is 1 or 2,
when n is 2, then it is also possible for $R_1$ to form with the phenyl group at the meta and para positions a dioxolane group

or a 1,4 dioxane group,
$R_2$ is selected from H and $NR_3R_4$,
$R_3$ is H and $R_4$ is phenyl optionally substituted with one or more substituents selected from OH, O-alkyl and Halo, or $R_3$ and $R_4$ together form a $(CH_2)_2O(CH_2)_2$ chain, that is to say that $NR_3R_4$ forms a morpholino group,
$R_5$ is selected from H, cyclic radical, O-alkyl, O-$(CH_2)_{n1}$-(cyclic radical), and $(CH_2)_{n1}$-(cyclic radical), $n_1$ being defined as previously, said cyclic radical being chosen from

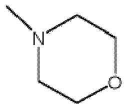 and 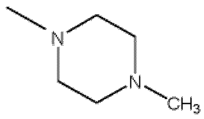,

$R_6$ is selected from H and Halo,
or a pharmaceutically acceptable salt and/or optical isomer, tautomer, or solvate thereof,
for use in the treatment of a disease selected from lymphomas and leukemia.

In the above general formula (I), unless specified otherwise:

Alkyl denotes a straight chain or branched group containing 1, 2, 3, 4, 5 or 6 carbon atoms. Examples of suitable alkyl radicals are methyl, ethyl, n-propyl, cyclopropyl, isopropyl, n-butyl, isobutyl, sec-butyl, tert-butyl, n-pentyl, cyclopentyl, isopentyl, n-hexyl, cyclohexyl, etc.

Alkyl is preferably a straight chain group comprising from 1 to 4 carbon atoms. Alkyl is preferably methyl.

The free bond on the phenyl group means that the phenyl may be substituted in the ortho, meta or para position and when n is 2, in two of the ortho, meta or para positions.
Halo means fluoro, chloro, bromo and iodo. A preferred Halo group for $R_1$ is fluoro or chloro, and more preferably chloro.
A preferred halo group for $R_2$ is chloro.
According to an advantageous embodiment of the invention, compounds for use according to the invention are of general formula (2):

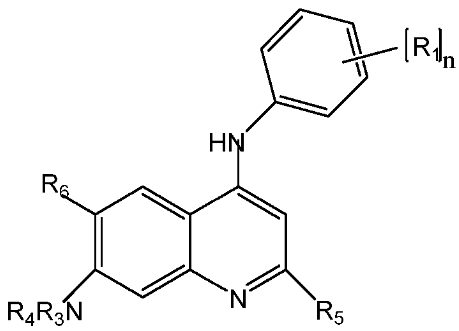
(2)

wherein $R_1$, $R_3$, $R_4$, $R_5$, $R_6$ and n are as defined in general formula (I).
According to an advantageous embodiment of the invention, compounds for use according to the invention are of general formula (3):

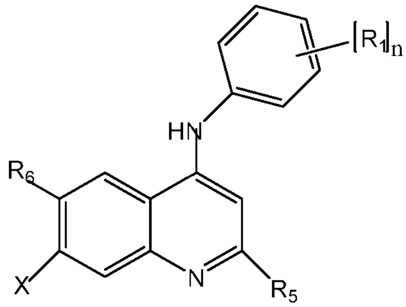
(3)

wherein $R_1$, $R_5$, $R_6$ and n are as defined in general formula (I) and X is a Halo chosen from Cl, F, Br and I. Preferably X is chloro.

According to another advantageous embodiment of the invention, compounds for use according to the invention are of general formula (3):

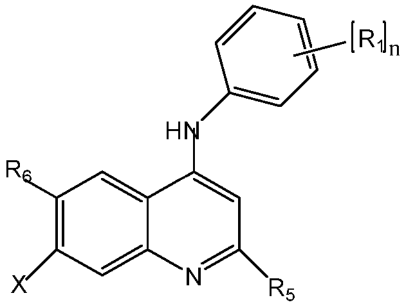

(3)

wherein $R_1$, $R_5$, $R_6$ and n are as defined in general formula (I) and X is a Halo chosen from Cl, F, Br and I, and with the proviso that $R_5$ is not H. Preferably X is chloro.

According to an embodiment of the invention, $R_1$ is selected from methyl ($CH_3$), fluoro (F), chloro (Cl), hydroxy (OH), methoxy ($OCH_3$), $CF_3$, $OCF_3$, $OCF_2H$ and $O(CH_2)_2OCH_3$, and when n is 2 then the substituents $R_1$ are identical or different.

According to another embodiment of the invention, when n is 2 then the two $R_1$ form with the phenyl group at the meta and para positions a dioxolane group or a 1,4 dioxane group.

According to an embodiment of the invention, in the compounds defined above, when $R_2$ is $NR_3R_4$, then $R_3$ is H and $R_4$ is a phenyl unsubstituted or substituted with one or two groups, identical or different, selected from OH, $OCH_3$ and Halo, Halo being preferably chloro.

According to another embodiment of the invention, in the compounds defined above, when $R_2$ is $NR_3R_4$, then $R_3$ and $R_4$ together form a $(CH_2)_2O(CH_2)_2$ chain, that is to say that $NR_3R_4$ forms a morpholino group.

According to an advantageous embodiment of the invention, compound (I) is selected from:

- *N*-(3,5-difluorophenyl)-7-chloroquinolin-4-amine (compound 1),
- *N*-(4-methylphenyl)-7-chloroquinolin-4-amine (compound 2),
- *N*-(3-trifluoromethylphenyl)-7-chloroquinolin-4-amine (compound 3),
- *N*-(2-methylphenyl)-7-chloroquinolin-4-amine (compound 4),
- *N*-(4-methoxyphenyl)-7-chloroquinolin-4-amine (compound 5),
- *N*-(3-methoxyphenyl)-7-chloroquinolin-4-amine (compound 6),
- *N*-(4-hydroxyphenyl)-7-chloroquinolin-4-amine (compound 7),
- *N*-(4-hydroxy-3-chlorophenyl)-7-chloroquinolin-4-amine (compound 8),
- *N*-(4-trifluoromethylphenyl)-7-chloroquinolin-4-amine (compound 9),
- *N*-(3,5-difluorophenyl)-7-morpholinoquinolin-4-amine (compound 10),
- *N*-(4-(difluoromethoxy)phenyl)-7-chloro-quinolin-4-amine (compound 34),
- *N*-(4-methoxy-3-(2-methoxyethoxy)phenyl)-7-chloro-quinolin-4-amine (compound 35),
- *N*-(4-trifluoromethoxyphenyl)-6-chloroquinolin-4-amine (compound 36),
- *N*-(2,4-dimethoxyphenyl)-6-chloroquinolin-4-amine (compound 37),
- *N*-(3,4-dimethoxyphenyl)-7-chloroquinolin-4-amine (compound 38),
- *N*-(2,4-dimethoxyphenyl)-7-chloroquinolin-4-amine (compound 39),
- *N*-(p-tolyl) 7-morpholinoquinolin-4-amine (compound 11),
- *N*-(o-tolyl)-7-morpholinoquinolin-4-amine (compound 12),
- *N*-(4-methoxyphenyl)-7-morpholinoquinolin-4-amine (compound 13),
- *N*-(3-methoxyphenyl)-7-morpholinoquinolin-4-amine (compound 14),
- *N*-(3-(trifluoromethyl)phenyl)-7-morpholinoquinolin-4-amine (compound 15),
- *N4, N7*-bis(4-methoxyphenyl)quinolin-4,7-diamine (compound 16),
- *N4, N7*-bis(3-methoxyphenyl)quinolin-4,7-diamine (compound 17),
- *N4*-(3,5-difluorophenyl)-N7-(4-methoxyphenyl)quinolin-4,7-diamine (compound 18),
- *N4*-(3,5-difluorophenyl)-N7-(3-chloro-4-hydroxyphenyl)quinolin-4,7-diamine (compound 19),
- *N4*-(p-tolyl)-N7-(3-methoxyphenyl)quinolin-4,7-diamine (compound 20),
- *N4*-(p-tolyl)-N7-(4-methoxyphenyl)quinolin-4,7-diamine (compound 21),
- *N4*-(3-(trifluoromethyl)phenyl)-N7-(4-methoxyphenyl)quinolin-4,7-diamine (compound 22),

- *N4*-(o-tolyl)-*N7*-(3-methoxyphenyl)quinolin-4,7-diamine (compound 23),
- *N4*-(4-methoxyphenyl)-N7-(3-methoxyphenyl)quinolin-4,7-diamine (compound 24),
- *N4*-(3-methoxyphenyl)-N7-(4-methoxyphenyl)quinolin-4,7-diamine (compound 25),
- *N4*-(3,5-difluorophenyl)-N7-(3-methoxyphenyl)quinolin-4,7-diamine (compound 26),
- *N*-(4-methoxyphenyl)-7-chloro-2-methoxy-quinolin-4-amine (compound 27),
- *N*-(3,4-dimethoxyphenyl)-7-chloro-2-morpholino-quinolin-4-amine (compound 28),
- *N*-(3,4-dimethoxyphenyl)-7-chloro-2-(4-methylpiperazin-1-yl)-quinolin-4-amine (compound 29),
- *N*-(4-methoxyphenyl)-7-chloro-2-morpholino-quinolin-4-amine (compound 30),
- *N*-(4-methoxyphenyl)-7-chloro-2-(4-methylpiperazin-1-yl)-quinolin-4-amine (compound 31),
- *N*-(3,4-dimethoxyphenyl)-7-chloro-2-(2-morpholinoethoxy)quinolin-4-amine compound 32),
- *N*-(3,4-dimethoxyphenyl)-7-chloro-2-methoxy-quinolin-4-amine (compound 33),
- *7-chloro-N*-(2,3-dihydroxybenzo[b][1,4]dioxin-6-yl)quinolin-4-amine (compound 40),
- *7-chloro-N*-(benzo[d][1,3]dioxol-5-yl)quinolin-4-amine (compound 41).

According to an advantageous embodiment of the invention, compound (I) is selected from:

- $N^4$, $N^7$-bis(4-methoxyphenyl)quinolin-4,7-diamine (compound 16),
- $N^4$, $N^7$-bis(3-methoxyphenyl)quinolin-4,7-diamine (compound 17),
- $N^4$-(3,5-difluorophenyl)-$N^7$-(4-methoxyphenyl)quinolin-4,7-diamine (compound 18),
- $N^4$-(3,5-difluorophenyl)-$N^7$-(3-chloro-4-hydroxyphenyl)quinolin-4,7-diamine (compound 19),
- $N^4$-(*p*-tolyl)-$N^7$-(3-methoxyphenyl)quinolin-4,7-diamine (compound 20),
- $N^4$-(*p*-tolyl)-$N^7$-(4-methoxyphenyl)quinolin-4,7-diamine (compound 21),
- $N^4$-(3-(trifluoromethyl)phenyl)-$N^7$-(4-methoxyphenyl)quinolin-4,7-diamine (compound 22),
- $N^4$-(*o*-tolyl)-$N^7$-(3-methoxyphenyl)quinolin-4,7-diamine (compound 23),
- $N^4$-(4-methoxyphenyl)-$N^7$-(3-methoxyphenyl)quinolin-4,7-diamine (compound 24),
- $N^4$-(3-methoxyphenyl)-$N^7$-(4-methoxyphenyl)quinolin-4,7-diamine (compound 25) and
- $N^4$-(3,5-difluorophenyl)-$N^7$-(3-methoxyphenyl)quinolin-4,7-diamine (compound 26).

According to an advantageous embodiment of the invention, compound (I) is selected from:

- *N*-(4-methoxyphenyl)-7-chloro-2-methoxy-quinolin-4-amine (compound 27),
- *N*-(3,4-dimethoxyphenyl)-7-chloro-2-morpholino-quinolin-4-amine (compound 28),
- *N*-(3,4-dimethoxyphenyl)-7-chloro-2-(4-methylpiperazin-1-yl)-quinolin-4-amine (compound 29),
- *N*-(4-methoxyphenyl)-7-chloro-2-morpholino-quinolin-4-amine (compound 30),
- *N*-(4-methoxyphenyl)-7-chloro-2-(4-methylpiperazin-1-yl)-quinolin-4-amine (compound 31),
- *N*-(3,4-dimethoxyphenyl)-7-chloro-2-(2-morpholinoethoxy)quinolin-4-amine (compound 32) and
- *N*-(3,4-dimethoxyphenyl)-7-chloro-2-methoxy-quinolin-4-amine (compound 33).

According to an advantageous embodiment of the invention, the halo group for $R_2$ is chloro (Cl).

The compounds of general formula (I) are more particularly the following:

- *N*-(4-methoxyphenyl)-7-chloroquinolin-4-amine (compound 5), and/or
- *N*-(3,4-dimethoxyphenyl)-7-chloroquinolin-4-amine (compound 38),

and/or the following:

- 7-chloro-*N*-(4-(2-methoxyethoxy)phenyl)quinolin-4-amine (compound 42) and,
- 7-chloro-*N*-(3-(hydroxy)-4-(methoxy)phenyl)quinolin-4-amine (also named 5-((7-chloroquinolin-4-yl)amino)-2-methoxyphenol) (compound 43).

The compounds of formula (I), their pharmaceutically acceptable salts and/or derived forms (optical isomers, tautomers, or solvates thereof), are valuable pharmaceutically active compounds suitable for the therapy and prophylaxis of lymphomas and/or leukemia. In particular, these compounds are useful for the therapy and prophylaxis of tumors of the lymphocyte lineages, for example B and T cell related leukemia (ALL/CLL).

The compounds of general formula (I) may be administered alone or in combination. They may also be administered in combination with one or more other drugs.

Generally, they will be administered as a formulation in association with one or more pharmaceutically acceptable excipients or carriers.

The term "excipient" or "carrier" is used herein to describe any ingredient other than the compound(s) of the invention. The choice of excipient will to a large extent depend on factors such as the particular mode of administration, the effect of the excipient on solubility and stability, and the nature of the dosage form.
Pharmaceutical compositions suitable for the delivery of compounds of the present invention and methods for their preparation will be readily apparent to those skilled in the art. Such compositions and methods for their preparation may be found, for example, in 'Remington's Pharmaceutical Sciences', 19th Edition (Mack Publishing Company, 1995).
Another aspect of the invention is thus a pharmaceutical composition for use in the treatment of a disease selected from lymphomas and leukemia comprising a compound of general formula (I) as defined above and optionally a pharmaceutically acceptable carrier.
According to another advantageous embodiment of the invention, the pharmaceutical composition for use as defined above may additionally comprise:

- at least one immunomodulatory compound, said immunomodulatory compound being preferably an immunomodulatory antibody, and even more preferably an antibody selected from an anti-PD1 antibody, an anti-CTLA4 antibody, an anti-PD-L1 antibody and a mixture of two or more thereof, and/or
- at least one another therapeutic agent.

According to another advantageous embodiment of the invention, the pharmaceutical composition for use as defined above may additionally comprise an anti-PD-L1 antibody.
The term "immunomodulatory compound" refers to a compound that modulates one or more of the components (e.g., immune cells, or subcellular factors, genes regulating immune components, cytokines, chemokines or such molecules) of a host's immune system.
Preferably, the immunomodulatory compound is an immunostimulatory agent. Immunomodulatory agents may include small molecules, peptides, polypeptides, fusion proteins, antibodies. Immunomodulatory antibodies are a promising class of anti-cancer therapies, due to their ability to promote a broad and sustained anti-cancer immune response in cancer patients.
Suitable examples of:

- anti-PD1 antibodies are nivolumab, pidizilumab, pembrolizumab and AMP-514,
- anti-CTLA4 antibodies are ipilimumab, tislelizumab
- anti PD-L1 antibodies are atezolizumab, durvalumab, avelumab, utomilumab and MPDL3280A.

The additional therapeutic agent(s) may also be (a) compound(s) of the formula (I), or a pharmaceutically acceptable salt, derived forms or compositions thereof, or one or more compounds known in the art for the treatment of lymphomas and leukemia.
For example, the additional therapeutic agent will be selected from a different class of therapeutic agents than those of the compounds of formula (I).
Suitable examples of other therapeutic agents which may be used in combination with the compound(s) of formula (I), or pharmaceutically acceptable salts or derived forms thereof, include:

- Anti-cancer agents used for the therapy of lymphomas such as dacarbazine,
- Nitrosourea alkylating agents, such as fotemustine,
- Anti PD1 fusion protein such as AMP-224,
- other immune checkpoint blocking agents or, in general, therapeutic agents based on immune approaches for treating lymphomas and/or leukemia,
- other therapeutic agents acting on the specific depletion/elimination of the malignant cells such as B and T-cell depleting antibodies ( Rituximab, Cetuximab, Alemtuzumab) or Chimeric Antigen Receptor (CAR) engineered T cells.

The invention more particularly relates to a pharmaceutical composition as defined above, as a combined preparation for simultaneous, separate or sequential use in the treatment of a disease selected from lymphomas and leukemia.
Inasmuch as it may desirable to administer a combination of active compounds for the purpose of treating a disease selected from lymphomas and leukemia, that two or more pharmaceutical compositions, at least one of which contains a compound in accordance with the invention, may conveniently be combined in the form of a kit suitable for co-administration of the compositions.
Thus, the kit of the invention comprises two or more separate pharmaceutical compositions, at least one of which contains a compound of formula (I) in accordance with the invention, and means for separately retaining said compositions, such as a container, divided bottle, or divided foil packet. An example of such a kit is the familiar blister pack used for

the packaging of tablets, capsules and the like.

The kit of the invention is particularly suitable for administering different dosage forms, for example parenteral, for administering the separate compositions at different dosage intervals, or for titrating the separate compositions against one another. To assist compliance, the kit typically comprises directions for administration and may be provided with a so-called memory aid.

As previously mentioned, the compounds of the formula (I), or pharmaceutically acceptable salts, derived forms or compositions thereof, can also be used as a combination with one or more additional therapeutic agents to be co-administered to a patient to obtain some particularly desired therapeutic end result in the treatment of lymphomas or leukemia.

Preferably, the compounds of the invention, either alone or in combination, are administered to patients at metastatic stage suffering from lymphomas or leukemia.

As used herein, the terms "co-administration", "co-administered" and "in combination with", referring to the compounds of formula (I) and one or more other therapeutic agents, is intended to mean, and does refer to and include the following: simultaneous administration of such combination of compound(s) of formula (I) and therapeutic agent(s) to a patient in need of treatment, when such components are formulated together into a single dosage form which releases said components at substantially the same time to said patient, substantially simultaneous administration of such combination of compound(s) of formula (I) and therapeutic agent(s) to a patient in need of treatment, when such components are formulated apart from each other into separate dosage forms which are taken at substantially the same time by said patient, whereupon said components are released at substantially the same time to said patient, sequential administration of such combination compound(s) of formula (I) and therapeutic agent(s) to a patient in need of treatment, when such components are formulated apart from each other into separate dosage forms which are taken at consecutive times by said patient with a significant time interval between each administration, whereupon said components are released at substantially different times to said patient; and sequential administration of such combination of compound(s) of formula (I) and therapeutic agent(s) to a patient in need of treatment, when such components are formulated together into a single dosage form which releases said components in a controlled manner whereupon they are concurrently, consecutively, and/or administered at the same and/or different times by said patient, where each part may be administered by either the same or different route.

Compounds of the invention may be administered as crystalline or amorphous products. They may be obtained, for example, as solid plugs, powders, or films by methods such as precipitation, crystallization, freeze-drying, spray drying, or evaporative drying. Microwave or radio frequency drying may be used for this purpose.

The compounds of the invention may be administered by any suitable route.

Thus, a compound of the invention may be formulated as a pharmaceutical composition for oral, buccal, intranasal, parenteral (e. g. intravenous, intramuscular or subcutaneous), topical or rectal administration or in a form suitable for administration by inhalation or insufflation.

For oral administration, the pharmaceutical composition may take the form of, for example, a tablet or capsule prepared by conventional means with a pharmaceutically acceptable excipient such as a binding agent (e. g., pregelatinized maize starch, polyvinylpyrrolidone or hydroxypropyl methylcellulose); filler (e. g., lactose, microcrystalline cellulose or calcium phosphate); lubricant (e. g., magnesium stearate, talc or silica); disintegrant (e. g., potato starch or sodium starch glycolate); or wetting agent (e. g., sodium lauryl sulphate).

The tablets may be coated by methods well known in the art. Liquid preparations for oral administration may take the form of a, for example, solution, syrup or suspension, or they may be presented as a dry product for constitution with water or other suitable vehicle before use. Such liquid preparations may be prepared by conventional means with a pharmaceutically acceptable additive such as a suspending agent (e. g., sorbitol syrup, methyl cellulose or hydrogenated edible fats); emulsifying agent (e. g., lecithin or acacia); non-aqueous vehicle (e. g., almond oil, oily esters or ethyl alcohol); and preservative (e. g., methyl or propyl p-hydroxybenzoates or sorbic acid).

For buccal administration, the composition may take the form of tablets or lozenges formulated in conventional manner. A compound of the present invention may also be formulated for sustained delivery according to methods well known to those of ordinary skill in the art. Examples of such formulations can be found in United States Patents 3,538, 214, 4,060, 598,4, 173,626, 3,119, 742, and 3,492, 397.

A compound of the invention may be formulated for parenteral administration by injection, including using conventional catheterization techniques or infusion. Formulations for injection may be presented in unit dosage form, e.g., in ampules or in multi-dose containers, with an added preservative. The compositions may take such forms as suspensions, solutions or emulsions in oily or aqueous vehicles, and may contain a formulating agent such as a suspending, stabilizing and/or dispersing agent. Alternatively, the active ingredient may be in powder form for reconstitution with a suitable vehicle, e. g., sterile pyrogen-free water, before use parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

For administration to human patients, the total daily dose of the compounds of the invention is typically in the range 0.001 mg to 5000 mg depending, of course, on the mode of administration. For example, an intravenous daily dose may only require from 0.001 mg to 40 mg. The total daily dose may be administered in single or divided doses and may, at the physician's discretion, fall outside of the typical range given herein.

These dosages are based on an average human subject having a weight of about 65 kg to 70 kg. The physician will readily be able to determine doses for subjects whose weight falls outside this range, such as infants and the elderly.

It is to be appreciated that all references herein to "treatment" include curative, palliative and prophylactic treatment.

The description, which follows, concerns the therapeutic applications to which the compounds of formula (I) may be put.

A still further aspect of the present disclosure also relates to the use of the compounds of formula (I), or pharmaceutically acceptable salts, derived forms or compositions thereof, for the manufacture of a drug having an anti-lymphoma or anti-leukemia activity.

In particular, the present disclosure concerns the use of the compounds of formula (I), or pharmaceutically acceptable salts, derived forms or compositions thereof, for the manufacture of a drug for the treatment of lymphomas or leukemia.

As a consequence, the present disclosure provides a particularly interesting method to treat a mammal, including a human being, with an effective amount of a compound of formula (I) or a pharmaceutically acceptable salt, derived form or composition thereof.

More precisely, the present disclosure provides a particularly interesting method for the treatment of a disease selected from a lymphoma and leukemia in a mammal, including a human being, comprising administering said mammal with an effective amount of a compound of formula (I), its pharmaceutically acceptable salts and/or derived forms.

According to another embodiment, the invention relates to a pharmaceutical composition for use in the treatment of cancer comprising a compound selected from:

- 7-chloro-*N*-(4-(2-methoxyethoxy)phenyl)quinolin-4-amine (compound 42) and,
- 7-chloro-*N*-(3-(hydroxy)-4-(methoxy)phenyl)quinolin-4-amine (also named 5-((7-chloroquinolin-4-yl)amino)-2-methoxyphenol) (compound 43); and
- at least one immunomodulatory compound, said immunomodulatory compound being preferably an immunomodulatory antibody, and even more preferably an antibody selected from an anti-PD1 antibody, an anti-CTLA4 antibody, an anti-PD-L1 antibody and a mixture of two or more thereof, preferably an anti-PD-L1 antibody and/or
- at least one another therapeutic agent.

By cancer is meant in particular, solid tumor cancer, and preferably those selected from melanoma, colon, lung, pancreas, kidney, Merkel carcinoma, squamous cell carcinoma, prostate, breast, bladder, leukemia and lymphomas.

In a particular embodiment, the cancer is melanoma.

Suitable examples of:

- anti-PD1 antibodies are nivolumab, pidizilumab, pembrolizumab and AMP-514,
- anti-CTLA4 antibodies are ipilimumab,
- anti PD-L1 antibodies are atezolizumab, durvalumab, avelumab, utomilumab and MPDL3280A.

The additional therapeutic agent(s) may also be (a) compound(s) of the formula (I), or a pharmaceutically acceptable salt, derived forms or compositions thereof, or one or more compounds known in the art for the treatment of cancer.

For example, the additional therapeutic agent will be selected from a different class of therapeutic agents than those of the compounds 42 and 43.

Suitable examples of other therapeutic agents which may be used in combination with the compound(s) 42 and 43 or pharmaceutically acceptable salts or derived forms thereof, include:

- Anti-cancer agents used for the therapy of lymphomas such as dacarbazine,
- Nitrosourea alkylating agents, such as fotemustine,
- BRAF inhibitors such as vemurafenib or dabrafenib,
- MEK inhibitors such as trametinib,
- Anti PD1 fusion protein such as AMP-224,
- other immune checkpoint blocking agents or, in general, therapeutic agents based on immune approaches for treating cancer.

The invention more particularly relates to a pharmaceutical composition as defined above, as a combined preparation for simultaneous, separate or sequential use in the treatment of cancer.

As previously mentioned, the compounds 42 and 43, or pharmaceutically acceptable salts, derived forms or compositions thereof, can also be used as a combination with one or more additional therapeutic agents to be co-administered to a patient to obtain some particularly desired therapeutic end result in the treatment of cancers, namely solid tumor cancer, and preferably those selected from melanoma, colon, lung, pancreas, kidney, Merkel carcinoma, squamous cell carcinoma, prostate, breast, bladder and lymphomas.

Preferably, the compounds 42 and 43, either alone or in combination, are administered to patients at metastatic stage suffering from cancers, namely solid tumor cancer, and preferably those selected from melanoma, colon, lung, pancreas, kidney, Merkel carcinoma, squamous cell carcinoma, prostate, breast, bladder and lymphomas.

A still further aspect of the present disclosure also relates to the use of the compounds 42 and 43, or pharmaceutically acceptable salts, derived forms or compositions thereof, in combination with:

- at least one immunomodulatory compound, said immunomodulatory compound being preferably an immunomodulatory antibody, and even more preferably an antibody selected from an anti-PD1 antibody, an anti-CTLA4 antibody, an anti-PD-L1 antibody and a mixture of two or more thereof, preferably an anti-PD-L1 antibody and/or
- at least one another therapeutic agent;

for the manufacture of a drug having an anti-cancer activity.

In particular, the present disclosure concerns the use of the compounds 42 and 43, or pharmaceutically acceptable salts, derived forms or compositions thereof, in combination with:

- at least one immunomodulatory compound, said immunomodulatory compound being preferably an immunomodulatory antibody, and even more preferably an antibody selected from an anti-PD1 antibody, an anti-CTLA4 antibody, an anti-PD-L1 antibody and a mixture of two or more thereof, preferably an anti-PD-L1 antibody and/or
- at least one another therapeutic agent;

for the manufacture of a drug for the treatment of cancers, namely solid tumor cancer, and preferably those selected from melanoma, colon, lung, pancreas, kidney, Merkel carcinoma, squamous cell carcinoma, prostate, breast, bladder and lymphomas.

As a consequence, the present disclosure provides a particularly interesting method to treat a mammal, including a human being, with an effective amount of a compound of 42 and/or 43 or a pharmaceutically acceptable salt, derived form or composition thereof in combination with:

- at least one immunomodulatory compound, said immunomodulatory compound being preferably an immunomodulatory antibody, and even more preferably an antibody selected from an anti-PD1 antibody, an anti-CTLA4 antibody, an anti-PD-L1 antibody and a mixture of two or more thereof, preferably an anti-PD-L1 antibody and/or
- at least one another therapeutic agent.

More precisely, the present disclosure provides a particularly interesting method for the treatment of a cancer in a mammal, including a human being, comprising administering said mammal with an effective amount of a compound 42 or 43, its pharmaceutically acceptable salts and/or derived forms in combination with:

- at least one immunomodulatory compound, said immunomodulatory compound being preferably an immunomodulatory antibody, and even more preferably an antibody selected from an anti-PD1 antibody, an anti-CTLA4 antibody, an anti-PD-L1 antibody and a mixture of two or more thereof, preferably an anti-PD-L1 antibody and/or
- at least one another therapeutic agent.

The compounds of the formula (I) may be prepared using conventional procedures such as by the following illustrative methods in which the various substituents are as previously defined for the compounds of the formula (I) unless otherwise stated.

Thus, compounds of general formula (I) can be prepared starting from the corresponding 4-chloro-7-haloquinoline by an aromatic nucleophilic substitution with appropriate amine, $R'_1$ being H or OH:

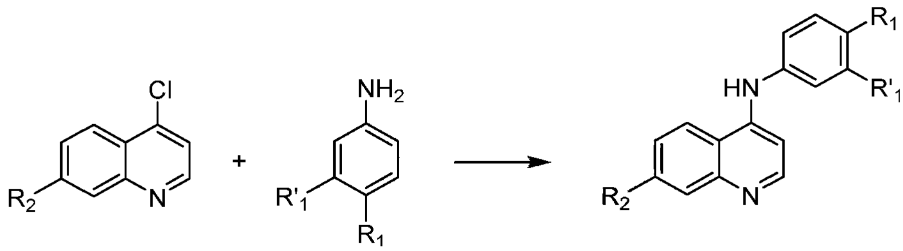

Compounds of general formula (1) wherein $K_2$ = fluoro or bromo, can be prepared starting from the corresponding 4-chloro-7-haloquinoline, according to the previously reported procedures (see for examples: Bioorg. Med. Chem. 2013, 21 (11), 3147-3153 ; J. Med. Chem., 2015, 58 (14), 5522-5537).

The procedure for preparing the compounds of the invention, wherein $R_5$ = $R_6$ = H and $R_2$ is chloro or $NR_3R_4$ comprises the following steps:

i) Reacting 4,7-dichloroquinoline with a compound of formula 4:

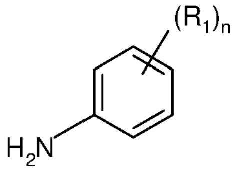

to obtain a compound of formula 3:

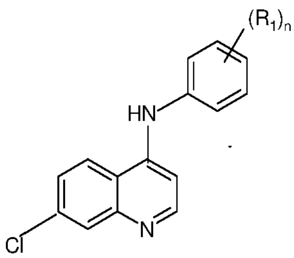

ii) If appropriate, reacting the compound of formula 3 with a compound of general formula 5: $HNR_3R_4$

iii) Obtaining the compound of formula (2), that is subsequently isolated and purified.

Step i) is performed in the conditions of an aromatic nucleophilic substitution in an acidic medium (Journal of the American Chemical Society, 1946, vol 68, 1807-1808).

Step ii) is performed in the conditions of a Buchwald-Hartwig coupling (Angewandte Chemie, International edition,1995, vol 34,1348-1350) in the presence of a catalyst, such as palladium and a base in a suitable solvent, e.g. Toluene, DME or dioxane.

Compounds of general formula 3 wherein $R_5$ = $R_6$ = H and X is fluoro or bromo, can be prepared starting from the corresponding 4-chloro-7-haloquinoline, according to the previously reported procedures (see for examples: Bioorg. Med. Chem. 2013, 21 (11), 3147-3153 ; J. Med. Chem., 2015, 58 (14), 5522-5537).

Compounds of general formula (I) wherein $R_2$ = $R_5$ = H and $R_6$ is Halo can be prepared starting from the corresponding 4-chloro-6-haloquinoline, according to the procedure described above for compound of formula (3).

Compounds of general formula (I) wherein $R_5 \neq$ H, $R_2$ = Cl and $R_6$ = H can be prepared by the following steps:

i) Reacting 2,4,7-trichloroquinoline with a compound of formula 4:

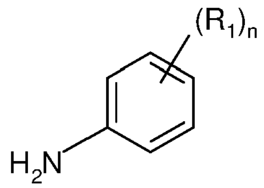

to obtain a compound of formula 6 :

ii) If appropriate, reacting the compound of formula 6 with a compound of general formula : $HNR_3R_4$ or O-alkyl.

Step i) is performed in the conditions of an aromatic substitution in basic medium in DMA (Tetrahedron Letters, 2013, vol 54, 6900-6904).

Step ii) is performed in the conditions of an aromatic substitution.

General and detailed procedures for the preparation of compounds according to the invention are mentioned in WO2018007648.

Pharmaceutically acceptable salts of the compounds of formula (I) include the acid addition and base salts thereof.

Suitable acid addition salts are formed from acids, which form non-toxic salts.

Examples include the acetate, aspartate, benzoate, besylate, bicarbonate/carbonate, bisulphate/sulphate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, saccharate, stearate, succinate, tartrate, tosylate and trifluoroacetate and xinafoate salts.

Suitable base salts are formed from bases, which form non-toxic salts.

Examples include the aluminium, arginine, benzathine, calcium, choline, diethylamine, diolamine, glycine, lysine, magnesium, meglumine, olamine, potassium, sodium, tromethamine and zinc salts. Hemisalts of acids and bases may also be formed, for example, hemisulphate and hemicalcium salts.

For a review on suitable salts, see "Handbook of Pharmaceutical Salts: Properties, Selection, and Use" by Stahl and Wermuth (Wiley-VCH, Weinheim, Germany, 2002).

Pharmaceutically acceptable salts of compounds of formula (I) may be prepared by one or more of three methods:

(i) by reacting the compound of formula (I) with the desired acid or base;

(ii) by removing an acid- or base-labile protecting group from a suitable precursor of the compound of formula (I) or by ring-opening a suitable cyclic precursor, for example, a lactone or lactam, using the desired acid or base; or

(iii) by converting one salt of the compound of formula (I) to another by reaction with an appropriate acid or base or by means of a suitable ion exchange column.

All three reactions are typically carried out in solution. The resulting salt may precipitate out and be collected by filtration or may be recovered by evaporation of the solvent. The degree of ionization in the resulting salt may vary from completely ionized to almost non-ionized.

The compounds of the invention may exist in both unsolvated and solvated forms.

The term "solvate" is used herein to describe a molecular complex comprising the compound of the invention and a stoichiometric amount of one or more pharmaceutically acceptable solvent molecules, for example, ethanol.

The term "hydrate" is employed when said solvent is water.

Included are complexes such as clathrates, drug-host inclusion complexes wherein, in contrast to the aforementioned solvates, the drug and host are present in stoichiometric or non-stoichiometric amounts. Also included are complexes of the drug containing two or more organic and/or inorganic components, which may be in stoichiometric or non-stoichiometric amounts. The resulting complexes may be ionized, partially ionized, or non-ionized. For a review of such complexes, see J Pharm Sci, 64 (8), 1269-1288 by Haleblian (August 1975).

Hereinafter all references to compounds of formula (I) include references to salts, solvates and complexes thereof and to solvates and complexes of salts thereof.

Compounds of formula (I) containing one or more asymmetric carbon atoms can exist as two or more stereoisomers. Included are all stereoisomers, geometric isomers and tautomeric forms of the compounds of formula (I), including compounds exhibiting more than one type of isomerism, and mixtures of one or more thereof.

Also included is acid addition or base salts wherein the counter ion is optically active, for example, D-lactate or L-lysine, or racemic, for example, DL-tartrate or DL-arginine.

Conventional techniques for the preparation/isolation of individual enantiomers include chiral synthesis from a suitable optically pure precursor or resolution of the racemate (or the racemate of a salt or derivative) using, for example, chiral high-pressure liquid chromatography (HPLC).

Alternatively, the racemate (or a racemic precursor) may be reacted with a suitable optically active compound, for example, an alcohol, or, in the case where the compound of formula (I) contains an acidic or basic moiety, an acid or base such as tartaric acid or 1-phenylethylamine. The resulting diastereomeric mixture may be separated by chromatography and/or fractional crystallization and one or both of the diastereoisomers converted to the corresponding pure enantiomer(s) by means well known to a skilled person.

Chiral compounds of the invention (and chiral precursors thereof) may be obtained in enantiomerically-enriched form using chromatography, typically HPLC (chiral columns), on an asymmetric resin with a mobile phase consisting of a hydrocarbon, typically heptane or hexane, containing from 0 to 50% by volume of isopropanol, typically from 2% to 20%, and from 0 to 5% by volume of an alkylamine, typically 0.1% diethylamine. For reverse HPLC $CH_3CN$ and $H_2O$, MeOH or iPrOH and $H_2O$ are used as solvents. Concentration of the eluate affords the enriched mixture.

Stereoisomeric conglomerates may be separated by conventional techniques known to those skilled in the art-see, for example, "Stereochemistry of Organic Compounds" by E. L. Eliel (Wiley, New York, 1994). "Chiral Separation Techniques". by G. Subramanian. John Wiley & Sons, 2008. "Preparative Enantioselective Chromatography" by G. B. Cox. Wiley, 2005.

Pharmaceutically acceptable solvates in accordance with the invention include those wherein the solvent of crystallization may be isotopically substituted, e.g. $D_2O$.

The following examples illustrate the preparation of the compounds of the formula (I) and their pharmacological properties.

**Figure 1:** mRNA levels of EZH2 detected by qPCR in A375 cancer cells incubated with compound 42, 43 (10μM) or DMSO (control) for 96h.

**Figure 2:** mRNA levels of p21 detected by qPCR in A375 cancer cells incubated with compound 42, 43 (10μM) or DMSO (control) for 96h.

**Figure 3:** IFN-γ levels detected by ELISA in supernatant of A375 cancer cells treated with compound 42, 43 (10μM) or DMSO (control) for 96 h.

**Figure 4:** Inhibition of the non-canonical NF-KB pathway or treatment with anti-*PD1 in vivo* in plck-GAPDH AITL mice increased their survival.

(A) Fold change mRNA expression levels of NIK in $CD4^+$-T cells from lymphoma developing plck-GAPDH mice as compared to $CD4^+$ T cells from wt spleens, set to 1 (means+/- SD, n=3; *p<0.05).

(B) Splenic lymphoma cells from plck-GAPDH mice were injected intravenously into recipient NSG mice (n= 10). Five lymphoma engrafted mice were treated with a NIK inhibitor (compound 5 according to the invention called $NIK^{inh-5}$) and 5 with vehicle on day 1 and 2 after tumor cell injection and then every other day. Comparison of survival curves between $NIK^{inh-5}$ treated and vehicle treated mice. Log-rank test; **p<0.01.

(C) Comparison of the percentage $CD4^+$-T cells in the spleens of $NIK^{inh-5}$ and vehicle treated groups and PD1 expression on the residual $CD4^+$ T cells (left panels). FACS plot is representative of n=5. Comparison of the percentage of $CD19^+$ B cells in the spleens of $NIK^{inh-S}$ and vehicle treated mice (right panels). FACS plot is representative of n=5. The ratio CD19/CD4 cells is shown (means+/- SD, n=5; **p<0.01).

**Figure 5:** Combining NIK Inhibition and anti-PD1 leads to survival of the plck-GAPDH AITL mouse model

(A) Fold change mRNA expression levels of NIK in $CD4^+$-T cells and GC B cells from lymphoma developing plck-GAPDH mice as compared to $CD4^+$ T cells from wt spleens, set to 1 (means+/- SD, n=3; ***p<0.001).

(B) Splenic lymphoma cells from plck-GAPDH mice were injected intravenously into recipient NSG mice (n= 18). Six lymphoma engrafted mice were treated with a NIK inhibitor, compound 38 according to the invention (called $NIK^{inh-32}$), 5 with vehicle and 7 with $NIK^{inh-32}$ + anti-PD1 on day 1 and 2 after tumor cell injection and then every other day. Comparison of survival curves between $NIK^{inh-32}$ treated, double-treated and vehicle treated mice is shown. Log-rank test ** p<0.01, * p<0.05.

(C) Spleen size of $NIK^{inh-32}$, double treated and vehicle treated mice at sacrifice; % B cells ($CD19^+$) and % T cells ($CD3^+$) in the spleen per total mononuclear cells for the indicated treatments groups at sacrifice (means+/- SD, n=4; *** p<0.001, **p<0.01, * p<0.05).

(D) Ratio of CD4/CD8 T cells and (E) % $PD1^{high}$ $CD4^+$ T cells in the spleen for the indicated treatments groups at sacrifice (means+/- SD, n=4; *** p<0.001, **p<0.01).

(F) Evaluation of the CD8 cytotoxicity in the spleens of $NIK^{inh-32}$, $NIK^{inh-32}$ + anti-PD1 and control treated NSG mice at sacrifice. Splenocytes were activated for 5h with PMA/ionomycin, then surface-stained for CD8 followed

by intracellular staining for IFNγ, Perforin and Gramzyme B (means+/- SD, n=3; *p<0.05, **p<0.01, ***p<0.001).
(G) Surface expression of the different TCR-β chains analyzed by FACS on the original engrafted plck-GAPDH $CD4^+$ T cells (plck-GAPDH) and on the CD4 T-cells in the spleens of $NIK^{inh}$, $NIK^{inh}$ + anti-PD1 treated NSG mice at sacrifice.
(H) mRNA expression levels of for canonical and non-canonical pathway genes and target genes in $CD4^+$ T cells for $NIK^{inh-32}$ + anti-PD1 double treated mice as compared to the vehicle treated group for which mRNA level was set to 1.

**Figure 6:** *in vitro* dose response study with compounds 5, 38, 42 and 43.
**Figure 7:** *in vivo* effect of compound 42 alone or in combination with an anti-PD-1 antibody.
**Figure 8:** NIK Inhibitors are specific for B cell lymphoma cell lines in which the non-canonical NF-kB pathway is activated. Effect of compound 38 treatment (10mM, 96Hrs) on the cell number of different Diffuse large B-cell lymphoma (DLBCL) B cell lines (U2932, KARPAS 422, SUDHL6, OCYL3). The cell number is normalized to the corresponding DMSO treated control cells.

## EXAMPLE 1: PREPARATION OF COMPOUNDS (I) OF THE INVENTION

Compounds 42 and 43 were prepared according to the procedure below.

• **7-chloro-N-(4-(2-methoxyethoxy)phenyl)quinolin-4-amine (compound 42)**

A mixture of 4,7-dichloroquinoline (1 mmol, 1eq) and 4-(2-methoxyethoxy)aniline (1 mmol, 1eq) in ethanol was subjected to microwave irradiation at 80°C for 1h. The mixture was cooled to room temperature before addition of ethyl acetate, the resulting precipitate was collected, wash with ethyl acetate and diethyl ether to afford pure product without any further purifications.

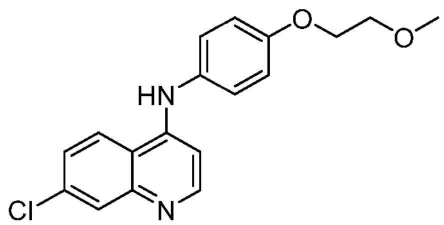

7-chloro-*N*-(4-(2-methoxyethoxy)phenyl)quinolin-4-amine
Chemical Formula: $C_{18}H_{17}ClN_2O_2$
Exact Mass: 328,0979
Molecular Weight: 328,7960

$^1$H NMR (400 MHz, DMSO-$d_6$) δ 14.79 (s, 1H), 11.13 (s, 1H), 8.87 (d, *J* = 9.1 Hz, 1H), 8.49 (d, *J* = 7.1 Hz, 1H), 8.18 (d, *J* = 2.2 Hz, 1H), 7.86 (dd, *J* = 9.1, 2.1 Hz, 1H), 7.40 (d, *J* = 8.9 Hz, 2H), 7.14 (d, *J* = 8.9 Hz, 2H), 6.65 (d, *J* = 7.0 Hz, 1H), 4.21 - 4.13 (m, 2H), 3.75 - 3.67 (m, 2H), 3.34 (s, 3H).
$^{13}$C NMR (101 MHz, DMSO) δ 157.2, 154.8, 142.6, 138.5, 137.76, 128.9, 126.7, 126.6, 125.6, 118.6, 115.2, 115.12, 99.5, 69.8, 66.7, 57.7.
**MS:** ESI (*m/z*): $[M+H]^+$ calcd for $C_{18}H_{17}ClN_2O_2$ 329.09 found 329.32
**HPLC:** Purity $\lambda_{254}$: 99.4%, tR: 3.54

• **7-chloro-N-(3-(hydroxy)-4-(methoxy)phenyl)quinolin-4-amine (compound 43)** (also named 5-((7-chloroquinolin-4-yl)amino)-2-methoxyphenol)

A mixture of 4,7-dichloroquinoline (1 mmol, 1eq) and 5-amino-2-methoxyphenol (1 mmol, 1eq) in ethanol was subjected to microwave irradiation at 80°C for 1h. The mixture was cooled to room temperature before addition of ethyl acetate, the resulting precipitate was collected, wash with ethyl acetate and diethyl ether to afford pure product without any further purifications.

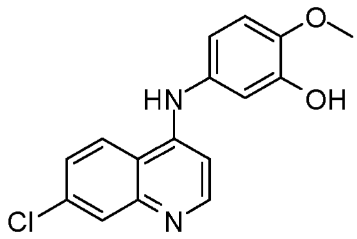

5-((7-chloroquinolin-4-yl)amino)-2-methoxyphenol
Chemical Formula: $C_{16}H_{13}ClN_2O_2$
Exact Mass: 300,0666
Molecular Weight: 300,7420

$^1$H NMR (400 MHz, DMSO-$d_6$) δ 14.41 (s, 1H), 10.88 (s, 1H), 9.56 (s, 1H), 8.76 (d, $J$ = 9.2 Hz, 1H), 8.49 (d, $J$ = 7.0 Hz, 1H), 8.10 (d, $J$ = 2.1 Hz, 1H), 7.86 (dd, $J$ = 9.1, 2.1 Hz, 1H), 7.09 (d, $J$ = 8.4 Hz, 1H), 6.91 - 6.81 (m, 2H), 6.72 (d, $J$ = 7.0 Hz, 1H), 3.84 (s, 3H).

$^{13}$C NMR (101 MHz, DMSO) δ 154.7, 147.1, 146.7, 142.5, 138.5, 137.7, 129.0, 126.6, 125.5, 118.6, 115.7, 115.2, 112.4, 112.3, 99.6, 55.3.

**MS:** ESI (*m/z*): [M+H]$^+$ calcd for $C_{16}H_{13}ClN_2O_2$ 301.06 found 301.14
**HPLC:** Purity $\lambda_{254}$: 99.0%, tR: 3.21

**EXAMPLE 2: *IN VITRO* ACTIVITY OF COMPOUNDS 42 AND 43 OF THE INVENTION**

**MATERIAL AND METHODS**

**Cancer cell proliferation assay (A375, A549, PC3, HT-29, MiaPaca-2 and MCF-7)**

The tested cancer cells are the following: human melanoma cells (A375), lung carcinoma cells (A549), prostate cancer cells (PC3), colon adenocarcinoma (HT29), pancreas carcinoma (MiaPaca 2) and breast carcinoma (MCF-7).

Cells were counted using a Malassez chamber. The average and standard deviation were calculated from triplicate experiments. Antiproliferative effects were evaluated by trypan blue exclusion assay. Briefly, cells were seeded into 12-well plate and grown in media supplemented with indicated compound at 1 or 10μM for 48h or 96h. Cells were washed with PBS and detached by trypsinization, collected and colored by trypan blue.

Dead and living cells were counted and proliferation was calculated as follow:

$$\text{Proliferation (\%)} = [(\text{treated cell number/untreated cells number})*100].$$

As a comparison, cell viability was also determined and calculated as follows: Viability (%) = 100 - [(dead cell number/total cells) *100].

Results obtained are summarized in **Tables 1a** (viability) and **1b** (proliferation).

**Melanocyte viability assay (MHN)**

Cell viability effects were evaluated by trypan blue exclusion assay. Briefly, cells were seeded into 12-well plate and grown in media supplemented with indicated compound at 10μM for 96h. Cells were washed with PBS and detached by trypsinization, collected and colored by trypan blue. Dead and living cells were counted and cell viability was calculated as follow: Viability (%) = 100 - [(dead cell number/total cells)* 100].

Results obtained are summarized in **Table 2.**

**Quantitative RT-PCR analysis of EZH2 and p21 gene expression**

Total RNA was isolated from cells using the RNAeasy minikit (Qiagen) according to the manufacturer's procedure. Reverse transcription was performed using the AMV reverse transcription system (Promega), and quantitative

PCR was performed with Power Sybr green (Applied Biosystems, Life Technologies, Grand Island, NY) following the manufacturer's instructions. The PCR was carried out on a Step One plus Real-Time PCR system (Applied Biosystems). All analyses were done in triplicate, and a melting curve analysis was performed to control for product quality and specificity. Expression levels were calculated using the comparative method of relative quantification, with SB34 as a normalizer. The data were analyzed for statistical significance using Student's t-test. The results are presented as the mean ± SEM relative to the control.

PCR primers for EZH2 (accession number NM004456.4) and p21(CDKN1A) (accession number NM078467.2) were obtained from primer bank or primer depot (http://pga.mgh.harvard.edu/primerbank/, https://primerdepot.nci.nih.gov), and their specificity was verified using primer blast (http://www.ncbi.nlm.nih.gov/tools/primer-blast/).

Results obtained are summarized in **Table 3** and **figures 1** (EZH2) and **2** (p21).

**ELISA measurement**

Supernatant from cells treated for 96 h was tested for human or mouse IFN-γ content via ELISA (peprotech, cat 900-k27 and 900-k98) kit following the manufacturer's instructions.

Results obtained are summarized in **Table 4** and **figure 3.**

**Quantitative senescence-associated beta-galactosidase assay**

The activity of senescence-associated β-galactosidase (SA-β-Gal) in cell extracts was quantified by measuring the cleavage of 4- methylumbelliferyl- 0 -D-galactopyrano side (4-MUG), which does not fluoresce until cleaved by the enzyme to generate the fluorophore 4-methylumbelliferone. The production of the fluorophore was monitored at an emission/excitation wavelength of 365/460 nm, as reported (Gary and Kindell, 2005)

Results obtained are summarized in **Table 5.**

**Microsomal stability (mouse liver microsomes)**

Microsomal stability was evaluated with mouse liver microsomes (0.5 mg/mL) and NADPH cofactor (1 mM) at 37°C. The percentage of remaining compound is determined at 5 min by LC-MS by measuring the area under the peak of compound on the chromatogram.

Results obtained are summarized in **Table 6.**

**RESULTS AND CONCLUSIONS**

The results obtained are summarized in Tables la, 1b, 2 to 6.

**Table 1a:** viability

| Tested compounds | Cancer Cell Viability (% a 10μM)* | | | | | |
|---|---|---|---|---|---|---|
| | A375 | A549 | PC3 | HT29 | MiaPaca-2 | MCF-7 |
| **Compound 42** | 99.3 | 99.7 | 98.9 | 97.3 | 100.0 | 97.2 |
| **Compound 43** | 95.8 | 99.8 | 94.6 | 85.8 | 97.6 | 99.6 |
| *Viability normalized to DMSO-treated control | | | | | | |

**Table 1b:** proliferation

| Tested compounds | Cancer Cell Proliferation (% à 10μM)* | | | | | |
|---|---|---|---|---|---|---|
| | A375 | A549 | PC3 | HT29 | MiaPaca-2 | MCF-7 |
| **Compound 42** | 6.9 | 19.5 | 14.1 | 3.5 | 7.8 | 11.9 |
| **Compound 43** | 30.5 | 51.1 | 55.0 | 15.6 | 32.4 | 29.5 |
| * Proliferation normalized to DMSO-treated control | | | | | | |

Table 1a shows that compounds 42 and 43 do not significantly affect the viability of treated cancer cells compared to untreated ones.

In the other hand, Table 1b shows that compounds 42 and 43, and more particularly compound 42, strongly reduce the proliferation of all tested cancer cells (human melanoma cells (A375), lung carcinoma cells (A549), prostate cancer cells (PC3), colon adenocarcinoma (HT29), pancreas carcinoma (MiaPaca 2) and breast carcinoma (MCF-7).
No decrease in viability is observed at early time points (up to 96h). The compounds provide a non-competitive inhibition on NIK leading to a strong selectivity without off target effects that is observed with competitive NIK inhibitors (9). By its action on EZH2, the inhibition of NIK leads to a demethylation of several key genes involved in cell cycle such as p21 and thus increases its expression. As a consequence, the proliferation of all treated cells tested so far is strongly reduced (from 55% to 3.5% depending on the compound and the cell line).

**Table 2:** melanocytes viability

| Tested compounds | Heathly Cell Viability (% a 10 μM)* |
|---|---|
| | MHN |
| Compound 42 | 99.0 |
| Compound 43 | 95.8 |
| *Viability normalized to DMSO-treated control | |

Table 2 shows that compounds 42 and 43 do not significantly affect the viability of human melanocytes.
NIK and the downstream target EZH2 are not expressed or at very low level in normal cells. Thus, the selective inhibition of NIK by the compounds of the invention do not alter normal cells and do not modify the viability of normal cells such as melanocytes.

**Table 3:** Quantitative RT-PCR analysis

| Tested **compounds** | qPCR* | |
|---|---|---|
| | EZH2 | p21 |
| | A375 | A375 |
| Compound 42 | 0.23 | 6.79 |
| Compound 43 | 0.62 | 3.08 |
| *mRNA (fold relative to DMSO-treated control) | | |

Table 3 shows that incubation of cells with compound 42 leads to a drastic decrease of mRNA expression of EZH2 as compared to incubation with a control compound (DMSO) **(figure 1).** Correspondingly treatment with compound 42 leads to a major increase of p21 mRNA expression **(figure 2).**
A more modest decrease of EZH2 mRNA expression was observed when cells were treated with compound 43, together with a significant increase of p21 mRNA expression.
Compounds 42 and 43 act by inhibiting NIK that regulates the non-canonical NF-kB pathway which in turn transcriptionally inhibits EZH2.
EZH2 is the central target as it decreases p21 by promoting its methylation and inhibits the transcription of IFN-γ by direct interaction on its promoter.
By decreasing EZH2 transcription, the compounds of the invention increase p21 and promote senescence. It thus induces a decrease in proliferation of treated cells. The immune activation is due to the induced secretion of IFN-γ by the treated cells.

**Table 4:** Elisa measurements

| Tested compounds | ELISA IFN-γ (A375)* |
|---|---|
| DMSO control | 0.17 |
| Compound 42 | 1.73 |
| Compound 43 | 0.41 |
| * IFN-γ concentration (ng/mL)/millions cells | |

Table 4 demonstrates that A375 cells treated with the compounds 42 and 43, and particularly compound 42, produce and secrete IFN-γ while almost no IFN-γ can be detected in untreated cells (**figure 3**).

EZH2 inhibits the production of IFN-γ by interfering directly on its promotor site. By downregulating EZH2 at its transcriptional level the compounds induce the transcription of IFN-γ and its production by the treated cancer cells. Table 4 shows a marked increase of the secretion of IFN-γ in the media by the treated cancer cells as compared to control. This local production of IFN-γ is crucial for attracting and activating the immune cells that will *in vivo* participate to the elimination of the cancer cells.

**Table 5** : Quantitative senescence-associated beta-galactosidase assay

| Tested compounds | % Senescence* |
|---|---|
| **Compound 42** | 645.9 |
| **Compound 43** | 1875.1 |
| *conversion rate (fold relative to DMSO-treated control) | |

The inhibition of the non-canonical NFkB pathway has been shown to decrease EZH2 and to restore a senescence program by decreasing the methylation of p21 and thus increasing its expression (9). Table 5 shows that the compounds of the invention (that inhibits the non-canonical NFkB pathway by selectively inhibiting NIK) induces a marked increase of senescence in the treated cells as compared to control. This increased senescence is in accordance with the increased expression of p21 shown in table 3.

**Table 6** : Microsomal stability

| Tested compounds | Microsomal stability, mouse liver (5min)* |
|---|---|
| **Compound 42** | 21% ± 3 |
| **Compound 43** | 41 % :t 3 |
| * Percentage of the remaining compound after 5 min of incubation with microsomes | |

Table 6 shows that compound 43 exhibits high microsomal stability compared to 42 (41% versus 21%). Microsomal stability serves as an *in vitro* assessment for the first path metabolisation of the drugs and is thus representative of an *in vitro* hepatic clearance, and an initial indicator for *in vivo* stability.

**EXAMPLE 3: *IN VITRO* ACTIVITY OF COMPOUNDS 5 AND 38 OF THE INVENTION**

**Material and methods**

**Mice**

**Generation of plck-GAPDH transgenic mice.**

The cDNA of human GAPDH fused to the V5 tag in the plasmid pcDNA3 was amplified by PCR and inserted in a plasmid under the control of the promoter of the kinase LCK (plck) upon BamHI digestion. The plck-GAPDH-V5 cassette was linearized from this plasmid and microinjected into the pronuclei of ovocytes from C57BL/6J mice that were subsequently implanted into gestating females (Service d'expérimentation animale et de Transgénèse (SEAT), CNRS, Villejuif, France).

Four transgenic founders (F35, F16, F07, F31) were selected and were bred by serially backcrossing with C57BL/6J mice for at least 12 generations before phenotypic analysis of the mice. C57BL/6J mice for breeding were obtained from ENVIGO (Cannat, France).

Genotyping of the plck-GAPDH mice was performed on genomic DNA isolated from a tail biopsy using the following couples of primers: S 1 and S2'. These primers were used with standard IL-2 primers.

Plck-p65$^{-/-}$ mice were generated by crossing pLck-Cre transgenic mice (Jackson Institute (n°cat #003802) with p65$^{flox/flox}$ mice provided by R. Schmidt (Algul et al., 2007) in our animal facility. Genotyping was performed using following primers: p651oxRV and p651$_0$xFW; LckCreRV and LckCreFW.

Plck-IKB$^{-/-}$ mice were generated by crossing plck-Cre transgenic mice with IKB $^{flox/flox}$ mice provided by R. Rupec (Rupec et al., 2005). Genotyping was performed using following primers: IkB-lox2-FW; IkB-Lox-2-RV.

Mice were bred and maintained under pathogen-free conditions at the local animal facility (C3M, INSERM U1065, Nice, France). All experimental procedures were carried out in compliance with protocols approved by the local ethical and experimentation committee (SBEA, Nice, France, autorization N° A06088014 and B0608820). At sacrifice, single cell suspensions were prepared from all the hematopoietic organs (spleen, lymph nodes, liver, blood and bone marrow) for further isolation and analysis.

**Western blot analysis**

Thymocytes of 5-week-old plck-GAPDH mice were isolated and lysed for western blot analysis. Mouse anti-V5 was purchased from Invitrogen (Carlsbad, CA, USA), rabbit anti-GAPDH from Abcam (Cambridge, UK) and mouse anti-Hsp90 (C45GS) from Cell signaling (Leiden, The Netherlands).

Antibodies against NF-κB1 (sc-166588X), NF-κB2 (sc-7386x), P65 (sc-8008x) and RelB (sc-166416x) were purchased from Santacruz (Heidelberg, Germany).

**Flow cytometry and antibodies**

For intracellular staining of Granzyme B, Perforin, INFγ, TNFα, and IL-10, IL-4 and IL-17 splenocytes were stimulated for 5 hours in PMA (phorbol 12-myristate-13-acetate; Sigma, France)/ionomycin (Sigma, France) in the presence of golgi-stop (BD Biosciences, Le Pont de Claix, France) and upon surface staining (anti-CD4 or anti-CD8) cells were fixed and permeabilized using the Cytofix/Cytoperm kit and protocol (BD Biosciences). Intracellular staining for V5tag and GAPDH was performed using the same Cytofix/Cytoperm kit.

All FACS analysis were performed using a MACSQUANT (Miltenyi Biotec, Paris, France).

**Immunohistochemistry analysis and antibodies**

Hematopoietic tissues were fixed in formaldehyde overnight and subsequently transferred into 70% ethanol and embedded in paraffin. Hematoxylin and eosin staining was performed on the paraffin embedded tissue sections to color the follicular center. Immuno-staining was performed using anti-mouse antibodies against CD3 (SP7) Spring (Roche, Boulogne-Billancourt, France), B220 (CD45R) BioRad (Marnes-la-Coquette France), KI67 (SP6), Labvision/Thermofisher. Follicular DCs were detected by anti-CD21 (Ep3093, Abcam, Paris, France).

**Detection of T cell clonality**

Clonality of T cells was determined by surface staining for TCR-β of the $CD4^+$ T cells in the tumors with antibodies directed against the different β-chains (Mouse Vβ TCR screening panel (FITC): β2, β3, β4, β5.1, β6, β7, β8.1, β8.3, β9, β10, β11, β12, β13, β14, β17; BD Biosciences).

**Tumor transplantation into NOD/SCIDγc-/- (NSG) mice**

Single cell suspensions from spleen or lymph nodes of old (> 18 months) plck-GAPDH mice with splenomegaly were injected intravenously (i.v.) into 6-8 week-old NSG mice (1-2E7 splenocytes per mouse). Recipient mice were sacrificed for evaluation when the mice became weak and/or splenomegaly could be detected (30 to 60 days after injection). The different hematopoietic organs (liver, spleen, blood) were isolated and single cell suspension prepared for FACS analysis (see below).

**Cytokine-chemokine array**

Mesenchymal lymph nodes from lymphoma developing plck-GAPDH mice and wt mice used as a control were stimulated for 4 hours with PMA and ionomycin in RPMI/10% FCS at a density of 5E6 cells/ml. Then the supernatant was tested for secreted cytokine and chemokine expression levels using the Proteome Profiler Mouse Cytokine Array Kit, Panel A (ARY006, R&D systems, Lille, France) according to manufacturer's instructions. Quantification was performed using Multigauge (Fuji Film, Tokyo, Japan).

**Transcriptomic analysis**

Plck-GAPDH versus wt mouse RNA seq data

Total RNA was extracted from splenocytes of plck-GAPDH mice, developing tumor and wt mice using the RNA

extraction kit (Qiagen, Valencia, CA, USA) according to manufacturer's instructions. Libraries were generated from 500ng of total RNAs using Truseq Stranded Total TNA kit (Illumina). Libraries were then quantified with Qubit dsDNA BR Assay kit (Invitrogen) and pooled. 4nM of this pool were loaded on a high output flow cell and sequenced on a NextSeq500 platform (Illumina) using 2x75bp paired-end chemistry. Illumina 2x75bp paired-end reads were mapped using STAR_2.4.0a versus mm10 Mus musculus build following Encode RNA-seq options. Feature Counts from subread package (release 1.5.0-p3-Linux-x86_64) was used with"--primary -g gene_name -p -s 1 -C" options to obtain a count matrix at the gene_name level for statistical analysis.

RNAseq data were quantified using RSEM sofware 1.2.25 (Li and Dewey, 2011).

The gene sets were chosen on the basis of know expression patterns in human and mice Tfh and both GC and post-GC B cell subsets. The results were analyzed using a 2-way hierarchical clustering.

The Sample Enrichment Scores (SES) were computed according to (Tosolini et al., 2016). by applying the open source software AutoCompare-SES (https://sites.google.com/site/fredsoftwares/products/autocompare_ses) with normalized settings" Each transcriptome was rank-ordered by decreasing expression levels and scored against a range of gene sets. The score for the enrichment of a gene set in a sample (SES) was defined as: SES = -log 10 (p), using p values from testing the alternative: "empirical distribution of the gene set in the transcriptome greater than uniform distribution". SES normalized scores are a % of the maximal theoretical score with the number of genes shares. SES normalized scores range between 0-100."

**AITL patient data**

Public raw data AITL transcriptomes using Affymetrix HGU133 Plus 2.0 microarrays were downloaded from GEO dataset (GSE58445 (Iqbal et al., 2014), GSE3526(Roth et al., 2006): GSE7307; https://www.ncbi.nlm.nih.gov/gds) and ArrayExpress (E-TABM-783 (de Leval et al., 2007); https://www.ebi.ac.uk/arrayexpress/), normalized together (RMA) and collapsed to HUGO gene symbols using chipset definition files.

For each gene, expression data were normalized with z-score methods and illustrated with heatmaps were using R software. Statistical differences were verified using an unpaired two-tailed Wilcoxon signed rank test versus the specified controls.

**Quantative PCRs**

$CD4^+$ T cells were isolated from the spleen of tumor bearing plck-GAPDH or wt C57/BL6 mice by incubation of total splenocytes an anti-mouse CD4-FITC antibody (Miltenyi) following by an incubation with anti-FITC microbeads according to manufacturer's instructions (Miltenyi). $CD19^+$ B cells were isolated using an anti-mouse CD19-FITC antibody and then following the same procedure as for $CD4^+$ cells.

Total RNA was extracted from cells using the RNA extraction kit (Qiagen, Valencia, CA, USA) according to manufacturer's instructions. After reverse transcription-PCR, the relative mRNA expression level of GAPDH, CXCL13, IL-21, Blimp, Bcl-6, RelA, cRel, GADD45B, E2F1, NF-KBia, HIF1a, LDHa, NF-kB2, RelB, EZH2, SP1, IRF4, ENPP2, NOD2, Autotaxin, 18S, (mouse) were obtained by real-time quantification PCR, using Sybergreen PCR Mix (Thermofisher Scientific, France) and the corresponding primers sets on the 7500 Fast and the Step One (Applied Biosystems) according to the manufacturer's instructions. All samples were normalized to 18S. the relative mRNA expression level of HIFla and LDHa were obtained by real-time quantification PCR, using the TaqMan PCR Master Mix (Eurogentec, Seraing, Belgium) and TaqMan assay primer set (Thermofisher; HIF1: Mm00468878_m1; LDHa: Mm00495282_g1) on the 7500 Fast and the Step One (Applied Biosystems) according to the manufacturer's instructions.

**PCR Amplification and Sanger Sequencing**

Mutations in the *RHOA, TET2, IDH2* and *DNMT3* allele were detected in plck-GAPDH tumor cells by PCR amplification using the KAPA HiFi HotStart ReadyMix PCR Kit (Kapa Biosystems, KK2601) and the following primers: RHOA-Fw and RHOA-Rv; TET2-Fw: and TET2-Rv; IDH2-Fw: and IDH2-Rv; DNMT3-Fw; DNMT3-Rv.

The PCR fragments were then sequenced by Sanger sequencing performed at Biofidal (Vaulx en Velin, France; www.biofidal.com).

**Treatment with NIK inhibitor**

Plck-GAPDH tumors were injected into 10 NSG mice (see above) and 5 were treated with a newly developed chemical NIK inhibitor (compound 5 according to the invention) or with Vehicle. IP injections were performed every other day with compound 5 (250 μg in 100 μl DMSO/Tween-20/Labrafil) or vehicle (100 μl DMSO/Tween-20/Labrafil). The second NIK inhibitor used (compound 38 according to the invention) was more soluble and IP injections were performed

every other day with compound 38 (250 μg in 100 μl NaCl 0,9%/mouse) or vehicle (100 μl NaCl 0,9%/mouse).

**Whole exome sequencing**

Genomic DNA was extracted from $CD4^+$ T cells as mentioned above. Paired-end DNA library was prepared according to the manufacturer's instructions (Agilent). The adapter-modified gDNA fragments were enriched by six cycles of PCR. Whole exome capture was carried out using Agilent's SureSelect Kit. After DNA quality evaluation, pooled samples were sequenced on Illunima Hiseq 2000.

Raw data were first filtered using an in-house quality control. Paired-end clean reads are aligned to the reference genome (UCSC hg19) using Burrows-Wheeler Aligner (BWA) software(Li and Durbin, 2009). If a read or reads pair is mapped to multiple positions, BWA will choose the most likely placement. While if two or more most-likely placements are present, BWA will choose any one randomly. Aligned reads were realigned to the genome. Genome Analysis Toolkit (GATK)(DePristo et al., 2011) was used to ignore those duplicates resulting from PCR amplification with Picard-tool. We utilized the Indelrealigner and RealignerTargetCreator in GATK do realignment around the indels according to GATK best practice. Furthermore, we performed base quality score recalibration with GATK to avoid system bias. After realignment to genome, we identified and filtered variants (SNP, INDELs) using GATK HaplotypeCaller and variantFiltration to guarantee meaningful analysis. Variants obtained from previous steps were compared based on the dbSNP(Sherry et al., 2001) and annotated with ANNOVAR(Wang et al., 2010).

**Anti-PD1 treatment**

Two different plck-GAPDH tumors expressing high levels of PD-1 on the $CD4^+$ T cells were injected into 10 NSG mice and the mice were treated with anti-PD1 antibody (clone PMP1-14, BioXcell; 125 μg in PBS/mouse) or an isotope control antibody daily for the first 2 days post-tumor injection, then every other day.

**Combined anti-PD1 and NIK inhibitor treatment**

The mice were injected with antibody (anti-PD1 or isotype control) in the morning and compound 38 according to the invention (injected in the evening) daily for the first 2 days plck-GAPDH post-tumor injection, then every other day. Same concentration as for single treatments was used.

Recipient mice were sacrificed at endpoint (> 10% weight loss or palpable splenomegaly). Single cell suspensions were prepared from the spleen, BM and liver for immunophenotypic FACS analysis.

**Statistics**

Statistical analyses were conducted using Microsoft excel 2013 and Prism software v6.0 (GraphPad Software, La Jolla, CA, USA). Results are indicated as means ± SD (standard deviation) in the figure legends unless otherwise. For statistical testing of significance, a student's T-test was used assuming equal variance and p-values are indicated in the figure legends. A p-value < 0.05 was considered to indicate statistical significance. Mice survival curves were evaluated using Log-rank test to determine significance. All flow cytometry data shown are representative of at least 3 reproduced experiments. Gene set enrichment analysis was performed as described above.

**Results**

**Overexpression of GAPDH exclusively in the T cell lineage does not affect early T cell differentiation**

Little is known about GADPH expression levels and its role in T cell differentiation and T cell cancers such as peripheral T cell lymphomas (PTCL). It is only recently emerging as a key factor in T cell survival and function (Balmer et al., 2016; Chang et al., 2013; Xu et al., 2016). Therefore, we generated a transgenic mouse model, in which we overexpressed GAPDH under the control of the T cell specific promoter, plck. A V5-tag was fused to GAPDH to distinguish it from the endogenous GAPDH enzyme expression and 4 founder animals were identified (data not shown). Overexpression of GAPDH was validated in the plck-GAPDH transgenic mice using an anti-V5 and anti-GAPDH antibody by western blot (data not shown). We confirmed exclusive and strong expression in the T cell lineage, inducing an overall increased GAPDH expression level in splenic T cells but not in B cells (data not shown). Thymocytes of 4-weekold mice were analyzed for GAPDH overexpression in the 4 founder animals and compared to wt. The GAPDHV5 expression was detected from the DN2 stage on (data not shown). All founders (F16, F31, F7 and F35) showed a significant overexpression in all the different thymic subpopulations compared to wt (data not shown). Higher GAPDHV5 expression was detected for F7 and F35, which led to an overall increase in GAPDH expression levels for these 2 founders (data not

shown). Interestingly, although GAPDH, as an inhibitor of CICD (Colell et al., 2007) might interfere with thymic development and selection, no significant differences in thymic subpopulations were revealed (data not shown).

**Aged plck-GAPDH mice develop a peripheral T cell lymphoma-like pathology**

Interestingly, the tumor frequency coincided with the GAPDH overexpression levels detected in the different founders (data not shown), since the high GAPDH expressing F07 and F35 mice all developed, while tumor frequency in F16 and F31 was much lower and took longer until onset of disease (data not shown). Therefore, we focused our study on F07 and F35. These plck-GAPDH mice developed no disease before 18 months and in some cases only after two years (data not shown).

First of all, the plck-GAPDH mice developed a generalized lymphadenopathy (data not shown), which was only detectable in an already advanced stage of the tumor development by sudden loss of weight. These mice developed in almost all of the cases a splenomegaly and at high frequency enlarged mesenchymal (MES) LN, hepatomegaly and less frequently, LNs at ectopic sites such as the kidney, heart or intestines (data not shown). The spleens and lymph nodes (LN) showed a greatly altered architecture and the follicular structures visible in the wt were destroyed in the plck-GAPDH mice, marked by large polymorphic infiltration of T cells and B cells (data not shown). Enlarged livers showed nodules formed by infiltrating lymphocytes (data not shown). All these pathological features together, point towards a peripheral T cell lymphoma (PTCL), characterized by generalized lymphopathy, splenomegaly, hepatomegaly, effected lymph node and follicular structures and extranodal manifestations. Even more remarkable was that aged plck-GAPDH mice developed also a typical skin rash and that their MES LNs and ectopic LNs showed a strong vascularization (data not shown). Moreover, 50% of plck-GAPDH mice developed ascites in the abdominal region (data not shown). Finally, the splenic tumors of the transgenic mice were characterized by a 2-fold higher cellularity of T cells and an increased ratio of $CD4^+$-T cells over $CD8^+$-T cells (data not shown).

The combination of all these histo-pathological symptoms suggests that the plck-GAPDH mice most probably develop PTCL disease.

**Plck-GAPDH mice develop a T cell lymphoma with a T1fh dominance.**

Since tumors in plck-GAPDH mice manifested a higher presence of CD4 over CD8 T cells, we characterized this $CD4^+$-T cell population. These tumor $CD4^+$-T cells were positive for programmed cell death 1 (PD1) and to a lesser extent for the chemokine receptor, CXCR5 (data not shown). The percentages of $CD4^+PD1^{high}CXCR5^+$ T follicular helper (Tfh)-like cell fraction was significantly higher in plck-GAPDH than in wt spleens but the increase in total $CD4^+PD1^{high}$ was even more pronounced reaching 80% (data not shown).

Additionally, the inducible T-cell co-stimulator (ICOS), a typical Tfh receptor assuring B cell interaction (Hu et al., 2011) was upregulated on the tumor $CD4^+$ T cells (data not shown). The $CD4^+$ plck-GAPDH neoplastic cells had an activated memory phenotype (data not shown) and showed a higher expression of the death receptor FAS (CD95) compared to $CD4^+$ wt T cells (data not shown), both characteristics of Tfh cells. Analysis of the proliferation marker, ki67, by FACS indicated that the majority of these $CD4^+$ T cells were strongly cycling in the plck-GAPDH spleens in contrast to wt spleens (data not shown) and this was confirmed by IHC ki67 stainings of the spleen (data not shown), indicating the aggressiveness of these malignant T cells. When we activated $CD4^+$ plck-GAPDH tumor cells by PMA/ionomycin, a strong expression of the inflammatory cytokines (TNF$\alpha$ and IFN$\gamma$) and the anti-inflammatory cytokine IL-10 was revealed (data not shown). The latter is known to promote B cell expansion activated by co-stimulatory signals. In contrast, IL-4 (Th2) and IL-17 (Th17) expression under these conditions was not increased in $CD4^+$ plck-GAPDH tumor cells (data not shown). Finally, we examined the $CD4^+$ T cells for TCR$\beta$ receptor expression. We clearly detected a TCR$\beta$ clonality or oligoclonality in the $CD4^+$ T cells from different plck-GAPDH tumor bearing mice confirming that they did develop a true T cell lymphoma (data not shown).

Summarizing the above results, the plck-GAPDH mice develop a PTCL with a pronounced Tfh-like phenotype.

**Plck-GAPDH Tfh-like lymphoma is associated with germinal center and plasma B cells**

Tfh reside in germinal centers (GCs) and normally interact with GC B cells, where they are critical for B cell survival, promoting Ig class switch recombination and somatic hypermutation, ultimately yielding plasma cells and memory B cells (Cildir et al., 2016). Indeed, B cell phenotyping of cells in the enlarged spleens and lymph nodes of the plck-GAPDH mice demonstrated significantly increased percentages of hyperplasic $FAS^+(CD95^+)$ $GL\text{-}7^+$ GC B cells compared to wt (data not shown B). Additionally, these $FAS^+GL\text{-}7^+$ GC B cells demonstrated increased levels of FAS and FAS-Ligand (CD178; data not shown).

Interaction with the Tfh cells is essential for the development of plasma B cells. Moreover, PD1 on Tfh regulates not only GC survival but also impacts on the PC differentiation (Hu et al., 2011; Xu et al., 2014). Identification of PC cells by

$CD138^+CD19^-B220^{low}$ surface expression showed that 30% of the plck-GAPDH mice showed more than 5% of PCs in their enlarged spleens or even lymph nodes varying from 6 to 80% of total mononuclear cells (data not shown).

Co-residence of Tfh and GC and plasma B cells in the plck-GAPDH tumors stress that this mouse model closely mimics a human Tfh PTCL cancer (Ahsanuddin et al., 2011; Nagoshi et al., 2013). This notion is even more reinforced by the presence of increased numbers of tumor-associated cells such as myeloid cells and in particular DCs in the enlarged plck-GAPDH spleens (data not shown) compared to wt mice. Of note, strong infiltration of the $CD4^+PD1^+$ Tfh cells and GC B cells in the bone marrow were confirmed in all tumors bearing plck-GAPDH mice (data not shown).

To confirm that the plck-GAPDH mouse developed a rare T cell lymphoma we tempted to transfer the tumor cells from enlarged LN or spleens into NOD/SCID $\gamma c^{-/-}$(NSG) immunodeficient recipient mice (Traggiai et al., 2004). The engrafted NSG mice recapitulated all the disease characteristics identical to the plck-GAPDH donor mice: a strong increase of CD4 vs CD8 ratio (data not shown), high expression of PD1 on $CD4^+$ T cells, and the co-residence of GC B cells in the tumors (data not shown). For 3 different tumor bearing donor mice (plck-GAPDH 1, 2 and 3), we confirmed Tfh and GC B cells colonization of the spleen in 3 independent recipients per donor (data not shown). Moreover, the NSG recipient mice showed identical pathogenesis as the donor mice: splenomegaly, hepatomegaly and ascites in the peritoneal abdomen (data not shown). Finally, we also detected infiltration of Tfh and GC B cells in the BM of the recipient NSG mice (data not shown). Thus, engraftment of these tumors confirmed that plck-GAPDH mouse developed a malignant T cell lymphoma with conservation of the tumor microenvironment.

Co-residence of Tfh and GC, plasma B cells and tumor associated cells in the plck-GAPDH tumors stress that this mouse model strongly mimics a Tfh PTCL.

**Plck-GAPDH PTCLs have a gene expression profile resembling that of AITL patient PTCL**

To identify the human counterpart of the mPTCL developed by plck-GAPDH mice, we compared the gene expression profile (GEP) of our mPTCL to those of hPTCLs. Initially, we compared GEPs of murine splenic PTCLs versus normal wt splenocytes resulting in 471 genes that were found significantly upregulated, while 847 genes were downregulated in the GAPDH overexpressing mPTCLs. We performed a gene set enrichment analysis (GSEA) for a Tfh-upregulated gene set, based on previous studies (de Leval et al., 2007; Jain et al., 2015). These Tfh signature genes (e.g. FOS, IL-21, PD1, LIF1) were significantly upregulated and enriched in the murine lymphomas as compared to wt (data not shown). This Tfh signature in our plck-GAPHD lymphoma $CD4^+$ T cells was already suggested by their immuno-phenotype ($CD4^+CXCR5^+ICOS^+PD1^{high}$, data not shown). To further validate the Tfh characteristic, we confirmed the upregulated expression of the chemokine CXCL13, which is critical for GC B cell recruitment and subsequent activation, IL-21, 'a helper cytokine' produced by Tfh cells that stimulates B to plasma B cell differentiation and Bcl-6, a Tfh master regulator, in mPTCL isolated $CD4^+$ T as compared to wt $CD4^+$ splenocytes (data not shown). In contrast Blimp, an antagonist regulator of Tfh cell differentiation was not upregulated (Johnston et al., 2009).

Additionally, GEA for a GC-plasma B cell signature (Recaldin and Fear, 2016) showed a remarkable upregulation for GC-enriched genes in mPTCLs (AICDA, PDL1, IRF4, CCL4, FAS, BAX) while CCR7 downregulation in GCs upon Tfh commitment was also confirmed (data not shown). However, XBP1, a key regulator of PC development, was also upregulated in the mPTCLs indicating that we have a mixed GC and PC B-cell signature.

One of most prominent human PTCL with dominance of Tfh cells associated with GC/plasma B cells is AITL, a rare devastating cancer (Federico et al., 2013). The described pathological (data not shown) and immunophenotypic (data not shown) characteristics of the plck-GAPDH mPTCL is matching in great detail hAITL (data not shown).

Indeed, when we compared the GEP data of LNs from > 30 AITL patients to healthy LN. As expected, we found increased expression of CXCR5, PD1, CXCL13, BCL6, ICOS and IL-21 confirming the Tfh neoplastic expression pattern in our AITL patient cohorts (data not shown), which is copied in the plck-GAPDH mPTCLs (data not shown). The GC B cell signatures in patients were underlined by increased FAS, BAX, AICDA, PDL1, IRF4, CCL4 expression and plasma B cell signature by XBP1 and CD138 expression in some AITL patients (data not shown), which also matched the plck-GAPDH mPTCLs (data not shown). To further comfort that our mouse developed AITL pathology, we decided to compare the cytokine and chemokine expression profiles found in AITL patients and plck-GAPDH mouse lymph nodes (data not shown). AITL is characterized by strong autoimmune features and inflammatory signals (de Leval et al., 2007) identical to those found in plck-GAPDH such as increased IFN$\gamma$, IL1R$\alpha$, IL-6 and TNF$\alpha$ (data not shown). We confirmed that by quantification of secreted cytokines and chemokines in PMA/ionomycin stimulated plck-GAPDH mPTCL. Increased levels of inflammatory (IFN$\gamma$, IL1Ralpha$\alpha$, IL-6 and TNF$\alpha$) and anti-inflammatory cytokines (IL-10, IL-6) were comparable to an AITL gene enriched inflammation signature (data not shown). In AITL, Tfh and GC B interaction is based on cytokine-chemokine cross-talks. The co-existence of Tfh and B cells in the plck-GAPDH could be explained by the presence of multiple chemokines attracting T cells, B cells but also multiple other immune cells such as DCs, macrophages and monocytes to the same microenvironment as found in AITL (Mourad et al., 2008). GEP of mPTCL versus wt splenocytes revealed an upregulated expression of multiple cytokines and chemokines (data not shown). This expression pattern of secreted chemokines and cytokines (data not shown) corresponded to those of AITL patient LN (data not shown).

Moreover, our mPTCL clearly showed a prominent upregulation of humoral immune response genes and genes related to vascularization and cell cycle entry (data not shown) confirming again the match between mPTCL and AITL characteristic features (hypergammaglobulinemia in 50-80% of the patients and increased tumor vascularization; (de Leval et al., 2010; de Leval et al., 2007).

Since we obtained this mPTCL by overexpressing GAPDH in the T cell lineage of our plck-GAPDH mouse model, we confirmed by GEA that the glycolytic enzyme GAPDH was still strongly overexpressed in the mPTCLs (data not shown). Detailed FACS analysis confirmed that the Tfh lymphoma $CD4^+$ T cells overexpressed GAPDH compared to aged matched wt animals (data not shown). Strikingly, we confirmed from GEA data also significant overexpression of GAPDH in AITL patient LN versus healthy LN (data not shown), strongly suggesting that a dysregulation of the GAPDH expression may be involved in the development and maintenance of this pathology. Strikingly, we revealed also *RHOA* mutations in our murine plck-GAPDH AITL disease model though we did not observe the *RHOA* G17V mutation (data not shown) typically found in AITL patients. Two mutations in the effector domain of *RHOA* in all 15 different tumors analyzed were found: one point mutation at position 37 (T37M), which by itself results in loss of RhoA binding to GTP, and an INDEL frameshift mutation consisting of an insertion of 5 bp at the codon 44, after which the reading frame is changed completely. Interestingly, the position 37 (T37A) in *RHOA* was also found mutated in gastric cancer and follicular lymphoma (Kakiuchi et al., 2014). The *RHOA* effector domain was also found mutated in Burkitt lymphoma (Rohde et al., 2014)(data not shown). Whole exome sequencing of the plck-GAPDH Tfh $CD4^+$ cells uncovered mutations in epigenetic modifiers such as *IDH2, DNMT3A* and *TET2.* This is in agreement with the frequent mutations of these genes in AITL patient cells (Cairns et al., 2012; Couronne et al., 2012; Lemonnier et al., 2012; Odejide et al., 2014; Quivoron et al., 2011). Moreover, frequently mutated genes in AITL patient tumors, involved in TCR-signaling and JAK/STAT-signaling were also found mutated in the different plck-GAPDH lymphoma $CD4^+$ T cells, again underlining the resemblance with AITL patients (Vallois et al., 2016).

**Plck-GAPDH develop an AITL-like PTCL via a GAPDH-dependent activation of the NF-κB pathway.**

Constitutive activation of the NF-κB signaling has been observed in various tumors including lymphomas and leukemia, which rely on NF-κB for their proliferation and survival (Imbert and Peyron, 2017). Additionally, we have shown that GAPDH enhances B lymphoma aggressiveness via activation of NF-KB (Chiche et al., 2015). Moreover, our gene expression profiling data showed that many NF-KB target genes are upregulated in our plck-GAPDH tumors such as INFγ, ICOS, IL-6, CCL5, CCL2, CCL4, CXCL9, 10 and 11, IgG heavy chains, CD40, CD44, ICAM-1 and IRF4 (non-extensive list). In agreement with (Chiche et al., 2015) we confirmed in 4-week old thymocytes from plck-GAPDH mice overexpressing GAPDH (data not shown), an upregulation of the NF-κB1 components of the canonical pathway (data not shown) and its target genes (GADD45, NF-KBIA, E2F1, HIF1a, LDHA).

GEA for a NF-KB gene signature (Gerondakis et al., 2014) showed an upregulation of the NF-KB canonical pathway but also a remarkable upregulation of the NF-κB non-canonical pathway and target genes in the plck-GAPDH mPTCL splenocytes versus wt (data not shown). Further Q-PCR analysis of isolated $CD4^+$-cells from mPTCL confirmed a mild upregulation of the classical pathway target gene NF-κBia (3-fold; data not shown) but a much stronger upregulation of the non-canonical pathway gene RelB and target genes EZH2 and IRF4 (data not shown).

To further confirm the equivalence of our plck-GAPDH mPTCL to hAITL pathology, we compared the mPTCL NF-κB signature to that of AITL patients. We detected also in AITL a strong activation of the non-canonical NF-κB pathway characterized by increased relB expression and strong upregulation of the non-canonical target genes EZH2 and IRF4, while other non-canonical targets, SP-1 and ENPP2, were not or slightly upregulated, equivalent to the mPTCL.

Finally, we aged plck-$p65^{-/-}$ mice, unable to activate the canonical NF-KB pathway in T cells, and plck-$I\kappa B^{-/-}$ mice, characterized by a constitutive activation of the NF-KB pathway specifically in the T cell compartment. At 18 months only the $I\kappa B^{-/-}$ mice developed a Tfh T cell lymphoma characterized by expression of CXCR5 and PD1 on $CD4^+$-T cells and GC B cell markers, CD95 and GL-7 on $CD19^+$-cells. They also showed an increased CD4/CD8 T-cell ratio and higher GAPDH expression levels. All these features are identical to mPTCL developed by plck-GAPDH mice (data not shown).

This indicates that GAPDH-induced constitutive activation of the NF-KB pathway in T cells results in a dominant Tfh PTCL equivalent to AITL, underlined by a shift towards activation of the NF-KB non-canonical pathway in the tumor cells.

**Plck-GAPDH mouse developing AITL respond to non-canonical NF-κB inhibition and anti-PD1 treatment**

We confirmed a strong activation of the non-canonical NF-KB pathway in mPTCL developed by plck-GAPDH mice. Activation of this pathway depends on the activation of the NF-KB inducing kinase (NIK) and IKKa phosphorylation (Xiao et al., 2001). Notably, NIK was also reported to be required for hT-cell lymphoma survival through NF-KB activation (Odqvist et al., 2013). Moreover, GC B cells, also express high levels of NIK to sustain their survival (Cildir et al., 2016; Mesin et al., 2016; Yamada et al., 2000). Therefore, NIK could represent an important therapeutic target in these types of AITL cancers. We therefore first confirmed a 9-fold and 3-fold increased NIK expression in our mPTCL $CD4^+$-T cells and

CD19$^+$-GC B cells, respectively, versus healthy counterparts (Figure 4A and data now shown).

We developed the NIK inhibitor compound 5 according to the invention (NIK$^{inh-5}$) and confirmed its activity *in vitro* (patent :PCT/EP2017/067306). A first plck-GAPDH tumor was engrafted in NSG mice as established before (data not shown). The NIK$^{inh-5}$ treated group showed significant increased survival as compared to the control group (Figure 4B). At sacrifice the former group showed that the spleen was completely devoid of GC B cells but Tfh CD4$^+$-T cell were still detected and expressed high levels of PD1 (Figure 4C). It is now clear that PD1 and its ligand PDL-1 play a central role in down-modulation of anti-tumor immunity by dampening T-cell effector functions. Indeed, use of blocking antibodies against PD1 or PDL-1 is emerging as an effective therapy reversing this cancer immunosuppression (Topalian et al., 2016). PD1 overexpression was indeed characteristic for CD4$^+$ Tfh cells in our plck-GAPDH AITL-like tumors (data not shown). Therefore, we evaluated an anti-PD1 immunotherapy. Donor mPTCL with characteristic Tfh phenotype and GC B cell invasion consisted of CD4 Tfh, CD8 T cells and GC B cells expressing the PDL-1 ligand (data not shown) at high levels meaning that anti-PD1 treatment may not only disrupt T-T cell interaction but also T-B cell interaction, known as a driver of T and B cell proliferation in this lymphoma. Importantly, PD1 was highly expressed on CD4$^+$ but not on CD8$^+$ T cells in the mPTCL (data not shown).

Two different plck-GAPDH tumors were engrafted in NSG mice and treated either with isotype or with anti-PD1 mAbs (data not shown). We detected for all the anti-PD1 treated engrafted NSG mice, a significantly increased survival as compared to the control animals. Two mice survived long-term upon continuous treatment (data not shown). In both *in vivo* experiments a strong depletion of CD4$^+$ T cells was detected in the spleen while CD8$^+$ cells were unaffected (data not shown) resulting in an inversed CD4/CD8 ratio (data not shown). This is in agreement with low PD1 expression on mPTCL CD8$^+$-cells as compared to the CD4$^+$ cells (data not shown). Macroscopically, the liver showed upon anti-PD1 treatment no white spots of immune cell infiltrates as seen for the isotype mAb treated controls (data not shown). In contrast, the percentage of B cells in the spleen was markedly increased upon anti-PD1 treatment but the percentage of GC B cells was reduced as compared to control mice (data not shown). Exactly the same CD4, CD8 T and B cell distribution was confirmed in the BM and the liver (data not shown). This was due to anti-PD1 treatment mediated reversion of immunosuppression as we confirmed increased production of INF$\gamma$, granzyme B and perforin by cytotoxic CD8$^+$ T cells (data not shown).

We first confirmed a 9-fold and 3-fold increased NIK expression in the mPTCL CD4$^+$-T cells and CD19$^+$-GC B cells, respectively, versus healthy counterparts (Figure 5A). Therefore, NIK could represent an important therapeutic target in AITL cancers. Additionally, PD1 and its ligand PDL-1 play a central role in down-modulation of anti-tumor immunity by dampening T-cell effector functions. Indeed, the use of blocking antibodies against PD1 or PDL-1 is emerging as an effective therapy reversing this cancer immunosuppression (Topalian et al., 2016). Therefore, we evaluated treatment with a novel NIK inhibitor alone or in combination with an anti-PD1 immunotherapy in our AITL preclinical model.

Since no specific NIK inhibitors are currently available, we developed the NIK inhibitor COMPONENT 38 (NIK$^{inh32}$), characterized by a potent anti-NIK activity but also high solubility, increasing the availability of the drug *in vivo* (patent PCT/EP2017/067306). We performed single treatment with this NIK$^{inh}$ and double treatment with anti-PD1 and NIK$^{inh}$. Both NIK$^{inh}$ single treatment or combined with anti-PD1 resulted in significant increased survival of up to 60% and 70% of the plck-GAPDH tumor engrafted mice, respectively (Figure 5B). At sacrifice, we detected a significant decrease in spleen size for the single and double treated mice compared to controls (Figure 5C). For both treatment conditions, we confirmed a highly significant decrease in B cells in the spleen while reduction in T cells was more prominent for the NIK$^{inh}$ single treatment than in combination with anti-PD1 (Figure 5C). However, both treatment regimens confirmed a strong reduction in CD4$^+$-T cells (Figure 5C,D), which in the case of double treatment was depleted completely for the Tfh PD1$^{high}$ population, while in NIK$^{inh}$ single treated mice a residual subpopulation of PD1$^{high}$CD4$^+$-cells was detected (Figure 5E). The CD4$^+$ TCR-Beta clonality for Beta 7 detected in the initial tumor was not detected as predominant in the NIK$^{inh}$ and anti-PD1 treated mice (Figure 5G). Additionally, in residual CD4$^+$-T cells upon combined NIK$^{inh}$ and anti-PD1 treatment a reduced expression EZH2 was confirmed compared to controls, demonstrating the efficacy of NIK$^{inh}$ to inhibit the non-canonical NF-$\kappa$B pathway *in vivo* in the tumoral Tfh cells (Figure 5H). In the double treated group, anti-PD1 mediated reversion of immunosuppression was confirmed by increased production of IFN$\gamma$, granzyme B and perforin by cytotoxic CD8$^+$-cells while the more potent NIK$^{inh}$ was primarily capable of increasing CD8 IFN$\gamma$ release (Figure 5F).

Summarizing, potent targeted inhibition of NIK, a key component of the non-canonical NF-KB pathway, alone or in combination with anti-PD1 immunotherapy increased survival of plck-GAPDH AITL tumor bearing mice by 60 to 70%, while single anti-PD1 immunotherapy was less effective. These results support that drugs targeting the non-canonical pathway represent a potential new treatment option for AITL.

**EXEMPLE 4: *IN VITRO* ACTIVITY OF COMPOUNDS 5, 38, 42 AND 43 OF THE INVENTION**

The melanoma cell line A375 was treated during 96h with graded concentration of compounds 5, 38, 42 and 43. At the end of the culture period, the cell concentration was determined.

Results demonstrate an inhibition of A375 proliferation starting around 5mM of compounds 5, 38, 42 and 43 (see Figure 6).

The IC50s are as follows:

- Compound 5: 1.33 μM;
- Compound 38: 0.56 μM;
- Compound 42: 0.42 μM; and
- Compound 43: 1.83 μM.

**EXAMPLE *5: IN VIVO* ACTIVITY OF COMPOUND 42 OF THE INVENTION**

B9 melanoma cells were administrated subcutaneously.
Compound 42 was administrated IP once every day at 50mg/kg. The anti-PD1 compound (BE0146-clone RMP1-14) was administered IP once every day at 10 mg/kg.
Administration of treatment was made when tumors are visible (between 50 and 100mm$^3$). The results are shown in Figure 7.

**EXAMPLE 6: *IN VITRO* ACTIVITY OF COMPOUND 38 OF THE INVENTION**

**Material and methods**

B cell lymphoma proliferation assay

The tested cancer cells are the following: the human Diffuse large B-cell lymphoma (DLBCL) cell lines: Karpas-422, SUDHL-6, U2932 and Oxyl3.
Cells were counted using a flow cytometer (MACQUANT, Miltenyi). 2E5 cells were seeded in 2 ml RPMI-GlutamaX supplemented with 10% FCS at start of the proliferation assay in a 6-well plate supplemented with the NIK Inhibitor (compound 38) at 10 μM for 96h. The cell numbers at 96h were then determined by counting using a flow cytometer. We distinguished dead and living cells using DAPI staining.
Proliferation was calculated as follows:

$$\text{Proliferation (\%)} = \text{(Treated cell number/untreated cells)* 100.}$$

The average and standard deviation were determined from 3 independent experiments.

**Results**

We compared the effects of NIK Inhibitor, compound 38, on the human Diffuse large B-cell lymphoma (DLBCL) B cell lines (U2932, KARPAS 422, SUDHL6, OCYL3) for their proliferation. Compared with controls (DMSO), the treatment with compound 38 had a strong inhibitory effect on the proliferation of the B cell lymphoma cancer cell lines (Figure 8) without affecting the cell viability (data not shown).

**REFERENCES:**

Throughout this application, various references describe the state of the art to which this invention pertains.

1. J. Fu, I. J. Malm, D. K. Kadayakkara, H. Levitsky, D. Pardoll, Y. J. Kim, Preclinical evidence that PD1 blockade cooperates with cancer vaccine TEGVAX to elicit regression of established tumors. Cancer research 74, 4042-4052 (2014).
2. T. Bald, J. Landsberg, D. Lopez-Ramos, M. Renn, N. Glodde, P. Jansen, E. Gaffal, J. Steitz, R. Tolba, U. Kalinke, A. Limmer, G. Jonsson, M. Holzel, T. Tuting, Immune cell-poor melanomas benefit from PD-1 blockade after targeted type I IFN activation. Cancer discovery 4, 674-687 (2014).
3. Z. Guo, H. Wang, F. Meng, J. Li, S. Zhang, Combined Trabectedin and anti-PD1 antibody produces a synergistic antitumor effect in a murine model of ovarian cancer. J Transl Med 13, 247 (2015).
4. H. E. Teulings, J. Limpens, S. N. Jansen, A. H. Zwinderman, J. B. Reitsma, P. I. Spuls, R. M. Luiten, Vitiligo-like depigmentation in patients with stage III-IV melanoma receiving immunotherapy and its association with survival: a systematic review and meta-analysis. J Clin Oncol 33, 773-781 (2015).
5. H. E. Teulings, M. Overkamp, E. Ceylan, L. Nieuweboer-Krobotova, J. D. Bos, T. Nijsten, A. W. Wolkerstorfer, R. M. Luiten, J. P. van der Veen, Decreased risk of melanoma and nonmelanoma skin cancer in patients with vitiligo: a

survey among 1307 patients and their partners. Br J Dermatol 168, 162-171 (2013).

6. M. Rashighi, P. Agarwal, J. M. Richmond, T. H. Harris, K. Dresser, M. W. Su, Y. Zhou, A. Deng, C. A. Hunter, A. D. Luster, J. E. Harris, CXCL10 is critical for the progression and maintenance of depigmentation in a mouse model of vitiligo. Sci Transl Med 6, 223ra223 (2014).

7. G. M. De Donatis, E. L. Pape, A. Pierron, Y. Cheli, V. Hofman, P. Hofman, M. Allegra, K. Zahaf, P. Bahadoran, S. Rocchi, C. Bertolotto, R. Ballotti, T. Passeron, NF-kB2 induces senescence bypass in melanoma via a direct transcriptional activation of EZH2. Oncogene, (2015*).*

8. T. W. Kang, T. Yevsa, N. Woller, L. Hoenicke, T. Wuestefeld, D. Dauch, A. Hohmeyer, M. Gereke, R. Rudalska, A. Potapova, M. Iken, M. Vucur, S. Weiss, M. Heikenwalder, S. Khan, J. Gil, D. Bruder, M. Manns, P. Schirmacher, F. Tacke, M. Ott, T. Luedde, T. Longerich, S. Kubicka, L. Zender, Senescence surveillance of pre-malignant hepatocytes limits liver cancer development. Nature 479, 547-551 (2011).

9. De Donatis GM, Le Pape E, Pierron A, Cheli Y, Hofman V, Hofman P, Allegra M, Zahaf K, Bahadoran P, Rocchi S, Bertolotto C, Ballotti R, Passeron T. NF-kB2 induces senescence bypass in melanoma via a direct transcriptional activation of EZH2. Oncogene 35 (21): 2813 (2016 May).

10. Ahsanuddin, A. N., Brynes, R. K., and Li, S. (2011). Peripheral blood polyclonal plasmacytosis mimicking plasma cell leukemia in patients with angioimmunoblastic T-cell lymphoma: report of 3 cases and review of the literature. Int J Clin Exp Pathol 4, 416-420.

11. Algul, H., Treiber, M., Lesina, M., Nakhai, H., Saur, D., Geisler, F., Pfeifer, A., Paxian, S., and Schmid, R. M. (2007). Pancreas-specific RelA/p65 truncation increases susceptibility of acini to inflammation-associated cell death following cerulein pancreatitis. J Clin Invest 117, 1490-1501.

12. Bachmann, I. M., Halvorsen, O. J., Collett, K., Stefansson, I. M., Straume, O., Haukaas, S. A., Salvesen, H. B., Otte, A. P., and Akslen, L. A. (2006). EZH2 expression is associated with high proliferation rate and aggressive tumor subgroups in cutaneous melanoma and cancers of the endometrium, prostate, and breast. J Clin Oncol 24, 268-273.

13. Berg, T., Thoene, S., Yap, D., Wee, T., Schoeler, N., Rosten, P., Lim, E., Bilenky, M., Mungall, A. J., Oellerich, T., et al. (2014). A transgenic mouse model demonstrating the oncogenic role of mutations in the polycomb-group gene EZH2 in lymphomagenesis. Blood 123, 3914-3924.

14. Chen, Z., Yang, P., Li, W., He, F., Wei, J., Zhang, T., Zhong, J., Chen, H., and Cao, J. (2018). Expression of EZH2 is associated with poor outcome in colorectal cancer. Oncol Lett 15, 2953-2961.

15. Chiche, J., Pommier, S., Beneteau, M., Mondragon, L., Meynet, O., Zunino, B., Mouchotte, A., Verhoeyen, E., Guyot, M., Pages, G., et al. (2015). GAPDH enhances the aggressiveness and the vascularization of non-Hodgkin's B lymphomas via NF-kappaB-dependent induction of HIF-1alpha. Leukemia 29, 1163-1176.

16. Cildir, G., Low, K. C., and Tergaonkar, V. (2016). Noncanonical NF-kappaB Signaling in Health and Disease. Trends Mol Med 22, 414-429.

17. Colell, A., Green, D. R., and Ricci, J. E. (2009). Novel roles for GAPDH in cell death and carcinogenesis. Cell Death Differ 16, 1573-1581.

18. de Leval, L., Gisselbrecht, C., and Gaulard, P. (2010). Advances in the understanding and management of angioimmunoblastic T-cell lymphoma. Br J Haematol 148, 673-689.

19. Gaulard, P., and de Leval, L. (2014). The microenvironment in T-cell lymphomas: emerging themes. Semin Cancer Biol 24, 49-60.

20. Jacquin, M. A., Chiche, J., Zunino, B., Beneteau, M., Meynet, O., Pradelli, L. A., Marchetti, S., Cornille, A., Carles, M., and Ricci, J. E. (2013). GAPDH binds to active Akt, leading to Bcl-xL increase and escape from caspase-independent cell death. Cell Death Differ 20, 1043-1054.

21. Lemonnier, F., and Mak, T. W. (2017). Angioimmunoblastic T-cell lymphoma: more than a disease of T follicular helper cells. J Pathol 242, 387-390.

22. Morin, R. D., Johnson, N. A., Severson, T. M., Mungall, A. J., An, J., Goya, R., Paul, J. E., Boyle, M., Woolcock, B. W., Kuchenbauer, F., et al. (2010). Somatic mutations altering EZH2 (Tyr641) in follicular and diffuse large B-cell lymphomas of germinal-center origin. Nat Genet 42, 181-185.

23. Nagoshi, H., Kuroda, J., Kobayashi, T., Maegawa, S., Chinen, Y., Kiyota, M., Nakayama, R., Mizutani, S., Shimura, Y., Yamamoto-Sugitani, M., et al. (2013). Clinical manifestation of angioimmunoblastic T-cell lymphoma with exuberant plasmacytosis. Int J Hematol 98, 366-374.

24. Odqvist, L., Sanchez-Beato, M., Montes-Moreno, S., Martin-Sanchez, E., Pajares, R., Sanchez-Verde, L., Ortiz-Romero, P. L., Rodriguez, J., Rodriguez-Pinilla, S. M., Iniesta-Martinez, F., et al. (2013). NIK controls classical and alternative NF-kappaB activation and is necessary for the survival of human T-cell lymphoma cells. Clin Cancer Res 19, 2319-2330.

25. Pardoll, D. M. (2012). The blockade of immune checkpoints in cancer immunotherapy. Nat Rev Cancer 12, 252-264.

26. Postow, M. A., Callahan, M. K., and Wolchok, J. D. (2015). Immune Checkpoint Blockade in Cancer Therapy. J Clin Oncol 33, 1974-1982.

27. Sun, S. C. (2017). The non-canonical NF-kappaB pathway in immunity and inflammation. Nat Rev Immunol 17, 545-558.
28. Topalian, S. L., Drake, C. G., and Pardoll, D. M. (2012). Targeting the PD-1/B7-H1(PD-L1) pathway to activate anti-tumor immunity. Curr Opin Immunol 24, 207-212.

## Claims

**1.** A compound of general formula (I):

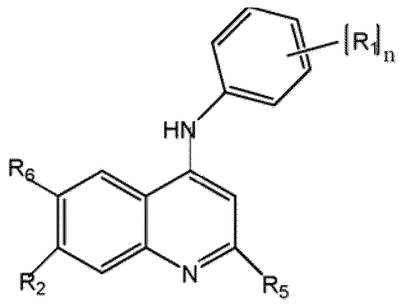

(I)

wherein:

n is 2 and each $R_1$ is selected from alkyl, Halo, OH, O-alkyl, $NH_2$, NH-alkyl, N-(alkyl)$_2$, S-alkyl, $CF_3$, $OCF_3$, $OCF_2H$ and $O(CH_2)_{n1}O(CH_2)_{n2}CH_3$, $n_1$ being 1 to 4 and $n_2$ being 0 to 3, Halo being selected from Cl, F, Br and I;
or n is 1 and $R_1$ is selected from alkyl containing 2 to 6 carbon atoms, I, O-alkyl containing 2 to 6 carbon atoms, $NH_2$, NH-alkyl, N-(alkyl)$_2$, S-alkyl, $OCF_3$, $OCF_2H$ and $O(CH_2)_{n1}O(CH_2)_{n2}CH_3$, $n_1$ being 1 to 4 and $n_2$ being 0 to 3;
when n is 2, then it is also possible for $R_1$ to form with the phenyl group at the meta and para positions a dioxolane group or a 1,4 dioxane group,
$R_2$ is selected from H, Halo and $NR_3R_4$, Halo being defined as previously,
$R_3$ is H and $R_4$ is phenyl optionally substituted with one or more substituents selected from OH, O-alkyl and Halo, or $R_3$ and $R_4$ together form a $(CH_2)_2O(CH_2)_2$ chain, that is to say that $NR_3R_4$ forms a morpholino group,
$R_5$ is selected from H, cyclic radical, O-alkyl, O-$(CH_2)_{n1}$-(cyclic radical), and $(CH_2)_{n1}$-(cyclic radical), $n_1$ being defined as previously, said cyclic radical being chosen from

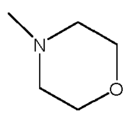 and 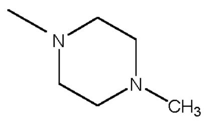,

$R_6$ is selected from H and Halo,
or a pharmaceutically acceptable salt and/or optical isomer, tautomer, or solvate thereof,
for use in the treatment of a disease selected from lymphomas and leukemia.

**2.** A compound for use according to claim 1 of general formula (2):

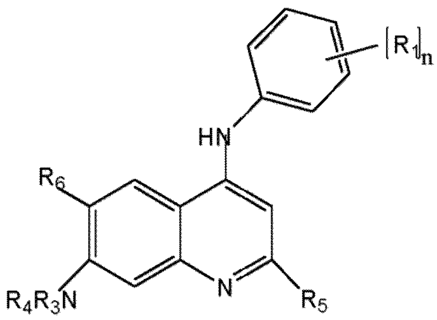

(2)

wherein $R_1$, $R_3$, $R_4$, $R_5$, $R_6$ and n are as defined in claim 1.

**3.** A compound for use according to claim 1 of general formula (3):

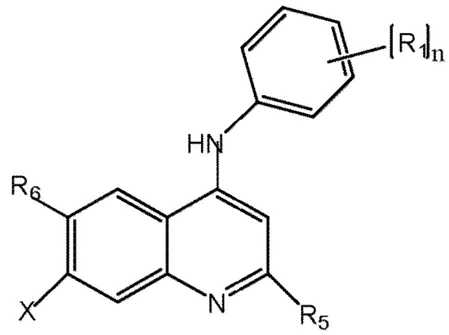

(3)

wherein $R_1$, $R_5$, $R_6$ and n are as defined in claim 1 and X is a Halo chosen from Cl, F, Br and I.

**4.** A compound for use according to any one of claims 1 to 3, wherein n is 2, wherein $R_1$ is selected from methyl ($CH_3$), fluoro (F), chloro (Cl), hydroxy (OH), methoxy ($OCH_3$), $CF_3$, $OCF_3$, $OCF_2H$ and $O(CH_2)_2OCH_3$, and wherein the substituents $R_1$ are identical or different.

**5.** A compound for use according to any one of claims 1 to 3, wherein when n is 2 then the two $R_1$ form with the phenyl group at the meta and para positions a dioxolane group or a 1,4 dioxane group.

**6.** A compound for use according to any one of claims 1, 2 and 4, wherein $R_3$ is H and $R_4$ is phenyl unsubstituted or substituted with one or two groups which are identical or different and which is/are selected from OH, $OCH_3$ and Halo, preferably chloro.

**7.** A compound of general formula (I): wherein:

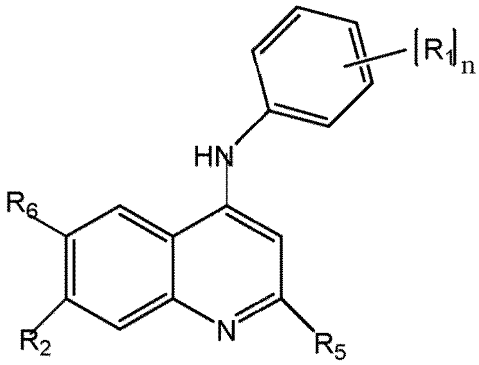

(I)

$R_1$ is selected from alkyl, Halo, OH, O-alkyl, $NH_2$, NH-alkyl, N-$(alkyl)_2$, S-alkyl, $CF_3$, $OCF_3$, $OCF_2H$ and $O(CH_2)_{n1}O(CH_2)_{n2}CH_3$, $n_1$ being 1 to 4 and $n_2$ being O to 3, Halo being selected from Cl, F, Br and I;
n is 1 or 2,
when n is 2, then it is also possible for $R_1$ to form with the phenyl group at the meta and para positions a dioxolane group or a 1,4 dioxane group,
$R_2$ is selected from H and $NR_3R_4$,
$R_3$ is H and $R_4$ is phenyl optionally substituted with one or more substituents selected from OH, O-alkyl and Halo, or $R_3$ and $R_4$ together form a $(CH_2)_2O(CH_2)_2$ chain, that is to say that $NR_3R_4$ forms a morpholino group,
$R_5$ is selected from H, cyclic radical, O-alkyl, O-$(CH_2)_{n1}$-(cyclic radical), and $(CH_2)_{n1}$-(cyclic radical), $n_1$ being defined as previously, said cyclic radical being chosen from

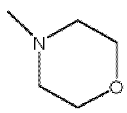 and 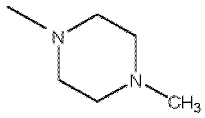,

$R_6$ is selected from H and Halo,
or a pharmaceutically acceptable salt and/or optical isomer, tautomer, or solvate thereof,
for use in the treatment of a disease selected from lymphomas and leukemia.

**8.** A compound for use according to claim 1, wherein said compound is selected from:

- *N*-(3,5-difluoropheny1)-7-chloroquinolin-4-amine,
- *N*-(4-hydroxy-3-chlorophenyl)-7-chloroquinolin-4-amine,
- *N*-(3,5-difluorophenyl)-7-morpholinoquinolin-4-amine,
- *N*-(4-(difluoromethoxy)phenyl)-7-chloro-quinolin-4-amine,
- *N*-(4-methoxy-3-(2-methoxyethoxy)phenyl)-7-chloro-quinolin-4-amine,
- *N*-(2,4-dimethoxyphenyl)-6-chloroquinolin-4-amine,
- *N*-(3,4-dimethoxyphenyl)-7-chloroquinolin-4-amine,
- *N*-(2,4-dimethoxyphenyl)-7-chloroquinolin-4-amine,
- *N4*-(3,5-difluorophenyl)-*N7*-(4-methoxyphenyl)quinolin-4,7-diamine,
- *N4*-(3,5-difluorophenyl)-*N7*-(3-chloro-4-hydroxyphenyl)quinolin-4,7-diamine,
- *N4*-(3,5-difluorophenyl)-*N7*-(3-methoxyphenyl)quinolin-4,7-diamine,
- *N*-(3,4-dimethoxyphenyl)-7-chloro-2-morpholino-quinolin-4-amine,
- *N*-(3,4-dimethoxyphenyl)-7-chloro-2-(4-methyl piperazin-1-yl)-quinolin-4-amine,
- *N*-(3,4-dimethoxyphenyl)-7-chloro-2-(2-morpholinoethoxy)quinolin-4-amine,
- *N*-(3, 4-dimethoxyphenyl)-7-chloro-2-methoxy-quinolin-4-amine,
- 7-chloro-*N*-(2,3-dihydroxybenzo[b][1,4]dioxin-6-yl)quinolin-4-amine, and
- 7-chloro-*N*-(benzo[d][1,3]dioxol-5-yl)quinolin-4-amine.

**9.** A compound for use according to any one of claims 1, 2, 4 and 6, wherein said compound is selected from:

- $N^4$-(3,5-difluorophenyl)- $N^7$- (4-methoxyphenyl)quinolin-4,7-diamine,
- $N^4$-(3,5-difluorophenyl)-$N^7$-(3-chloro-4-hydroxyphenyl)quinolin-4,7-diamine,
and
- $N^4$-(3,5-difluorophenyl)-$N^7$-(3-methoxyphenyl)quinolin-4,7-diamine.

**10.** A compound for use according to any one of claims 1, 3 and 4, wherein said compound is selected from:

- *N*-(3,4-dimethoxyphenyl)-7-chloro-2-morpholino-quinolin-4-amine,
- *N*-(3,4-dimethoxyphenyl)-7-chloro-2-(4-methylpiperazin-1-yl)-quinolin -4-amine,
- *N*-(3,4-dimethoxyphenyl)-7-chloro-2-(2-morpholinoethoxy)quinolin-4-amine and
- *N*-(3,4-dimethoxyphenyl)-7-chloro-2-methoxy-quinolin-4-amine.

**11.** A compound for use according to claim 1, wherein said compound is selected from:

- 7-chloro-N-(4-(2-methoxyethoxy)phenyl)quinolin-4-amine and
- 7-chloro-N-(3-(hydroxy)-4-(methoxy)phenyl)quinolin-4-amine.

**12.** A compound for use according to claim 7, wherein said compound is selected from:

- *N*-(4-trifluoromethoxyphenyl)-6-chloroquinolin-4-amine,
- *N*-(p-tolyl)-7-morpholinoquinolin-4-amine,
- *N*-(o-tolyl)-7-morpholinoquinolin-4-amine,
- *N*-(4-methoxyphenyl)-7-morpholinoquinolin-4-amine,
- *N*-(3-methoxyphenyl)-7-morpholinoquinolin-4-amine,
- *N-(*3-trifluoromethyl)phenyl)-7-morpholinoquinolin-4-amine,
- *N4,N7*-bis(4-methoxyphenyl)quinolin-4,7-diamine,
- *N4,N7*-bis(3-methoxyphenyl)quinolin-4,7-diamine,

- *N4*-(p-tolyl)-N7-(3-methoxyphenyl)quinolin-4,7-diamine,
- *N4*-(p-tolyl)-N7-(4-methoxyphenyl)quinolin-4,7-diamine,
- *N4*-(3-(trifluoromethyl)phenyl)-N7-(4-methoxyphenyl)quinolin-4,7-diamine,
- *N4-(*o-tolyl)-N7-(3-methoxyphenyl)quinolin-4,7-diamine,
- *N4-(*4-methoxyphenyl)-N7-(3-methoxyphenyl)quinolin-4,7-diamine, and
- *N4*-(3-methoxyphenyl)-N7-(4-methoxyphenyl)quinolin-4,7-diamine.

**13.** A pharmaceutical composition for use in the treatment of a disease selected from lymphomas and leukemia comprising:

- a compound as defined in any one of claims 1 to 12,
- optionally at least one pharmaceutically acceptable carrier.

**14.** A pharmaceutical composition for use according to claim 13, additionally comprising:

- at least one immunomodulatory compound, said immunomodulatory compound being preferably an immunomodulatory antibody, and even more preferably an antibody selected from an anti-PDl antibody, an anti-CTLA4 antibody, an anti-PD-LI antibody and a mixture of two or more thereof, and/or
- at least one other therapeutic agent.

**15.** A pharmaceutical composition for use according to claim 14, as a combined preparation for simultaneous, separate or sequential use.

## Patentansprüche

**1.** Verbindung der allgemeinen Formel (I):

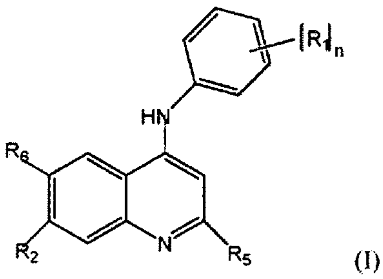

(I)

wobei:

n 2 ist und jedes $R_1$ aus Alkyl, Halo, OH, O-Alkyl, $NH_2$, NH-Alkyl, N-$(Alkyl)_2$, S-Alkyl, $CF_3$, $OCF_3$, $OCF_2H$ und $O(CH_2)_{n1}O(CH_2)_{n2}CH_3$ ausgewählt wird, wobei $n_1$ 1 bis 4 und $n_2$ 0 bis 3 ist, wobei Halo aus Cl, F, Br und I ausgewählt wird;
oder n 1 ist und $R_1$ aus Alkyl, das 2 bis 6 Kohlenstoffatome enthält, I, O-Alkyl, das 2 bis 6 Kohlenstoffatome enthält, $NH_2$, NH-Alkyl, N-$(Alkyl)_2$, S-Alkyl, $OCF_3$, $OCF_2H$ und $O(CH_2)_{n1}O(CH_2)_{n2}CH_3$ ausgewählt wird, wobei $n_1$ 1 bis 4 ist und $n_2$ 0 bis 3 ist;
wenn n 2 ist, dann ist es auch möglich, dass $R_1$ mit der Phenylgruppe an den Meta- und Parapositionen eine Dioxolangruppe oder eine 1,4-Dioxangruppe bildet,
$R_2$ aus H, Halo und $NR_3R_4$ ausgewählt wird, wird Halo wie zuvor definiert,
$R_3$ H ist und $R_4$ Phenyl ist, das optional mit einem oder mehreren Substituenten substituiert ist, die aus OH, O-Alkyl und Halo ausgewählt werden,
oder $R_3$ und $R_4$ zusammen eine $(CH_2)_2O(CH_2)_2$-Kette bilden, d. h., $NR_3R_4$ bildet eine Morpholinogruppe,
$R_5$ aus H, zyklischen Radikalen, O-Alkyl, O-$(CH_2)_{n1}$-(zyklischen Radikalen) und $(CH_2)_{n1}$-(zyklischen Radikalen) ausgewählt wird, wobei $n_1$ wie zuvor definiert ist, und das zyklische Radikal aus

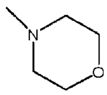 und 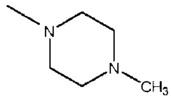,

ausgewählt wird,
$R_6$ aus H und Halo ausgewählt wird,
oder ein pharmazeutisch akzeptables Salz und/oder optisches Isomer, Tautomer oder Solvat davon zur Verwendung bei der Behandlung einer unter Lymphomen und Leukämie ausgewählten Krankheit.

2. Verbindung zur Verwendung nach Anspruch 1 der allgemeinen Formel (2):

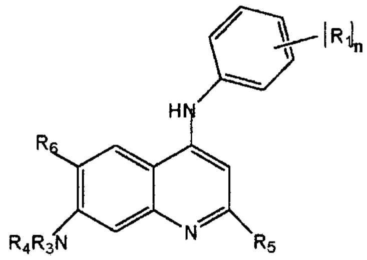
(2)

wobei $R_1$, $R_3$, $R_4$, $R_5$, $R_6$ und n wie in Anspruch 1 definiert sind.

3. Verbindung zur Verwendung nach Anspruch 1 der allgemeinen Formel (3):

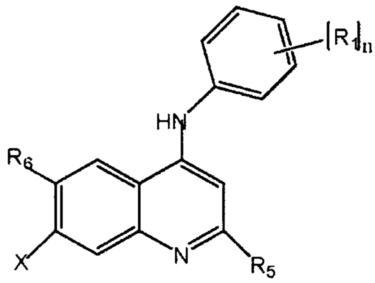
(3)

wobei $R_1$, $R_5$, $R_6$ und n wie in Anspruch 1 definiert sind und X ein Halo ist, der aus Cl, F, Br und I ausgewählt wird.

4. Verbindung zur Verwendung nach einem der Ansprüche 1 bis 3, wobei n 2 ist und $R_1$ aus Methyl ($CH_3$), Fluor (F), Chlor (Cl), Hydroxy (OH), Methoxy ($OCH_3$), $CF_3$, $OCF_3$, $OCF_2H$ und $O(CH_2)_2OCH_3$ ausgewählt wird, und wobei die Substituenten $R_1$ identisch oder unterschiedlich sind.

5. Verbindung zur Verwendung nach einem der Ansprüche 1 bis 3, wobei, wenn n 2 ist, die beiden $R_1$ mit der Phenylgruppe an der Meta- und Paraposition eine Dioxolangruppe oder eine 1,4-Dioxangruppe bilden.

6. Verbindung zur Verwendung nach einem der Ansprüche 1, 2 und 4, wobei $R_3$ H ist und $R_4$ Phenyl ist, das unsubstituiert oder mit einer oder zwei Gruppen substituiert ist, die identisch oder unterschiedlich sind und die aus OH, $OCH_3$ und Halo, vorzugsweise Chlor, ausgewählt werden.

7. Verbindung der allgemeinen Formel (I):

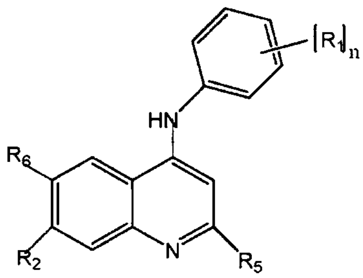

(I)

wobei:

$R_1$ aus Alkyl, Halo, OH, O-Alkyl, $NH_2$, NH-Alkyl, N-$(Alkyl)_2$, S-Alkyl, $CF_3$, $OCF_3$, $OCF_2H$ und $O(CH_2)_{n1}O(CH_2)_{n2}CH_3$ ausgewählt wird, wobei $n_1$ von 1 bis 4 und $n_2$ von 0 bis 3 reicht, wobei Halo aus Cl, F, Br und I ausgewählt wird;
n 1 oder 2 ist,
wenn n 2 ist, dann ist es auch möglich, dass $R_1$ mit der Phenylgruppe an den Meta-und Parapositionen eine Dioxolangruppe oder eine 1,4-Dioxangruppe bildet,
$R_2$ aus H und $NR_3R_4$ ausgewählt wird,
$R_3$ H ist und $R_4$ Phenyl ist, das optional mit einem oder mehreren Substituenten substituiert ist, die aus OH, O-Alkyl und Halo ausgewählt werden,
oder $R_3$ und $R_4$ zusammen eine $(CH_2)_2O(CH_2)_2$-Kette bilden, d. h., $NR_3R_4$ bildet eine Morpholinogruppe,
$R_5$ aus H, zyklischen Radikalen, O-Alkyl, O-$(CH_2)_{n1}$-(zyklischen Radikalen) und $(CH_2)_{n1}$-(zyklischen Radikalen) ausgewählt wird, wobei $n_1$ wie zuvor definiert ist, und das zyklische Radikal aus

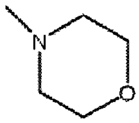 und 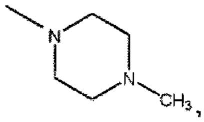,

ausgewählt wird,
$R_6$ aus H und Halo ausgewählt wird,
oder ein pharmazeutisch akzeptables Salz und/oder optisches Isomer, Tautomer oder Solvat davon zur Verwendung bei der Behandlung einer unter Lymphomen und Leukämie ausgewählten Krankheit.

**8.** Verbindung zur Verwendung nach Anspruch 1, wobei die Verbindung aus Folgendem ausgewählt wird:

- *N*-(3,5-Difluorphenyl)-7-chlorchinolin-4-amin,
- *N*-(4-Hydroxy-3-chlorphenyl)-7-chlorchinolin-4-amin,
- *N*-(3,5-Difluorphenyl)-7-morpholinochinolin-4-amin,
- *N*-(4-(Difluormethoxy)phenyl)-7-chlorchinolin-4-amin,
- *N*-(4-Methoxy-3-(2-methoxyethoxy)phenyl)-7-chlorchinolin-4-amin,
- *N*-(2,4-Dimethoxyphenyl)-6-chlorchinolin-4-amin,
- *N*-(3,4-Dimethoxyphenyl)-7-chlorchinolin-4-amin,
- *N*-(2,4-Dimethoxyphenyl)-7-chlorchinolin-4-amin,
- *N4*-(3,5-Difluorphenyl)-*N7*-(4-methoxyphenyl)chinolin-4,7-diamin,
- *N4*-(3,5-Difluorphenyl)-*N7*-(3-chlor-4-hydroxyphenyl)chinolin-4,7-diamin,
- *N4*-(3,5-Difluorphenyl)-*N7*-(3-methoxyphenyl)chinolin-4,7-diamin,
- *N*-(3,4-Dimethoxyphenyl)-7-chlor-2-morpholino-chinolin-4-amin,
- *N*-(3,4-Dimethoxyphenyl)-7-chlor-2-(4-methylpiperazin-1-yl)-chinolin-4-amin,
- *N*-(3,4-Dimethoxyphenyl)-7-chlor-2-(2-morpholinoethoxy)chinolin-4-amin,
- *N*-(3,4-Dimethoxyphenyl)-7-chlor-2-methoxy-chinolin-4-amin,
- 7-Chlor-*N*-(2,3-dihydroxybenzo[b][1,4]dioxin-6-yl)chinolin-4-amin, und
- 7-Chlor-*N*-(Benzo[d][1,3]dioxol-5-yl)chinolin-4-amin.

**9.** Verbindung zur Verwendung nach einem der Ansprüche 1, 2, 4 und 6, wobei die Verbindung aus Folgendem ausgewählt wird:

- $N^4$-(3,5-Difluorphenyl)-$N^7$-(4-methoxyphenyl)chinolin-4,7-diamin,
- $N^4$-(3,5-Difluorphenyl)-$N^7$-(3-chlor-4-hydroxyphenyl)chinolin-4,7-diamin, und
- $N^4$-(3,5-Difluorphenyl)-$N^7$-(3-Methoxyphenyl)chinolin-4,7-diamin.

**10.** Verbindung zur Verwendung nach einem der Ansprüche 1, 3 und 4, wobei die Verbindung aus Folgendem ausgewählt wird:

- *N*-(3,4-Dimethoxyphenyl)-7-chlor-2-morpholino-chinolin-4-amin,
- *N*-(3,4-Dimethoxyphenyl)-7-chlor-2-(4-methylpiperazin-1-yl)-chinolin-4-amin,
- *N*-(3,4-Dimethoxyphenyl)-7-chlor-2-(2-morpholinoethoxy)chinolin-4-amin und
- *N*-(3,4-Dimethoxyphenyl)-7-chlor-2-methoxy-chinolin-4-amin.

**11.** Verbindung zur Verwendung nach Anspruch 1, wobei die Verbindung aus Folgendem ausgewählt wird:

- 7-Chlor-N-(4-(2-methoxyethoxy)phenyl)chinolin-4-amin und
- 7-Chlor-N-(3-(Hydroxy)-4-(Methoxy)phenyl)chinolin-4-amin.

**12.** Verbindung zur Verwendung nach Anspruch 7, wobei die Verbindung aus Folgendem ausgewählt wird:

- *N*-(4-Trifluormethoxyphenyl)-6-chlorchinolin-4-amin,
- *N*-(p-Tolyl)-7-morpholinochinolin-4-amin,
- *N*-(o-Tolyl)-7-morpholinochinolin-4-amin,
- *N*-(4-Methoxyphenyl)-7-morpholinochinolin-4-amin,
- *N*-(3-Methoxyphenyl)-7-morpholinochinolin-4-amin,
- *N*-(3-Trifluormethyl)phenyl)-7-morpholinochinolin-4-amin,
- *N4,N7*-bis(4-Methoxyphenyl)chinolin-4,7-diamin,
- *N4,N7*-bis(3-Methoxyphenyl)chinolin-4,7-diamin,
- *N4*-(p-Tolyl)-N7-(3-methoxyphenyl)chinolin-4,7-diamin,
- *N4*-(p-Tolyl)-N7-(4-methoxyphenyl)chinolin-4,7-diamin,
- *N4*-(3-(Trifluormethyl)phenyl)-N7-(4-methoxyphenyl)chinolin-4,7-diamin,
- *N4*-(o-Tolyl)-N7-(3-methoxyphenyl)chinolin-4,7-diamin,
- *N4*-(4-Methoxyphenyl)-N7-(3-methoxyphenyl)chinolin-4,7-diamin, und
- *N4*-(3-Methoxyphenyl)-N7-(4-methoxyphenyl)chinolin-4,7-diamin.

**13.** Pharmazeutische Zusammensetzung zur Verwendung bei der Behandlung einer aus Lymphomen und Leukämien ausgewählten Krankheit, umfassend:

- eine Verbindung nach einem der Ansprüche 1 bis 12,
- optional mindestens einen pharmazeutisch akzeptablen Träger.

**14.** Pharmazeutische Zusammensetzung zur Verwendung nach Anspruch 13, die zusätzlich Folgendes umfasst:

- mindestens eine immunmodulierende Verbindung, wobei die immunmodulierende Verbindung vorzugsweise ein immunmodulierender Antikörper und noch bevorzugter ein Antikörper ist, der aus einem Anti-PD1-Antikörper, einem Anti-CTLA4-Antikörper, einem Anti-PD-L1-Antikörper und einer Mischung aus zwei oder mehr davon ausgewählt wird, und/oder
- mindestens ein weiteres therapeutisches Mittel.

**15.** Pharmazeutische Zusammensetzung zur Verwendung nach Anspruch 14, als kombinierte Zubereitung zur gleichzeitigen, separaten oder sequenziellen Verwendung.

**Revendications**

**1.** Composé de formule générale (I) :

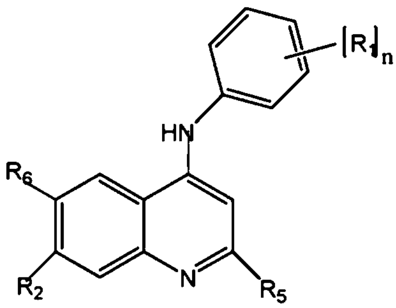

(I)

dans laquelle :

n vaut 2 et chaque $R_1$ est sélectionné parmi les groupes alkyle, Halo, OH, O-alkyle, $NH_2$, NH-alkyle, N-(alkyle)$_2$, S-alkyle, $CF_3$, $OCF_3$, $OCF_2H$ et $O(CH_2)_{n1}O(CH_2)_{n2}CH_3$, $n_1$ valant de 1 à 4 et $n_2$ valant de 0 à 3, Halo étant sélectionné parmi Cl, F, Br et I;
ou n vaut 1 et $R_1$ est sélectionné parmi les groupes alkyle comprenant de 2 à 6 atomes de carbone, I, O-alkyle comprenant de 2 à 6 atomes de carbone, $NH_2$, NH-alkyle, N-(alkyle)$_2$, S-alkyle, $OCF_3$, $OCF_2H$ et $O(CH_2)_{n1}O(CH_2)_{n2}CH_3$, $n_1$ valant de 1 à 4 et $n_2$ valant de 0 à 3;
quand n vaut 2, alors il est aussi possible pour $R_1$ de former avec le groupe phényle aux positions méta et para un groupe dioxolane ou un groupe 1,4 dioxane,
$R_2$ est sélectionné parmi H, Halo et $NR_3R_4$, Halo étant défini comme précédemment,
$R_3$ est H et $R_4$ un phényl optionnellement substitué par un ou plusieurs substituants sélectionnés parmi OH, O-alkyle et Halo,
ou $R_3$ et $R_4$ forment ensemble une chaine $(CH_2)_2O(CH_2)_2$, c'est-à-dire que $NR_3R_4$ forme un groupe morpholino,
$R_5$ est sélectionné parmi H, radical cyclique, O-alkyle, O-$(CH_2)_{n1}$-(radical cyclique), et $(CH_2)_{n1}$-(radical cyclique), $n_1$ étant défini comme précédemment, ledit radical cyclique étant sélectionné parmi

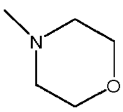 et 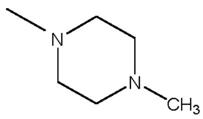,

$R_6$ est sélectionné parmi H et Halo,
ou un sel pharmaceutiquement acceptable et/ou un isomère optique, un tautomère ou un solvate de celui-ci, pour son utilisation dans le traitement d'une maladie sélectionnée parmi les lymphomes et la leucémie.

**2.** Composé pour son utilisation selon la revendication 1, de formule générale (2) :

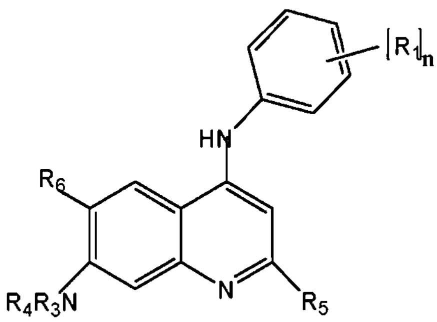

(2)

dans laquelle $R_1$, $R_3$, $R_4$, $R_5$, $R_6$ et n sont tels que définis à la revendication 1.

**3.** Composé pour son utilisation selon la revendication 1, de formule générale (3) :

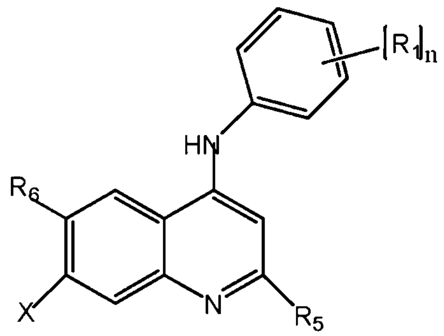

(3)

dans laquelle $R_1$, $R_5$, $R_6$ et n sont tels que définis à la revendication 1 et X est un Halo choisi parmi Cl, F, Br et I.

**4.** Composé pour son utilisation selon l'une quelconque des revendications 1 à 3, dans lequel n vaut 2, $R_1$ est sélectionné parmi méthyle ($CH_3$), fluoro (F), chloro (Cl), hydroxy (OH), méthoxy ($OCH_3$), $CF_3$, $OCF_3$, $OCF_2H$ et $O(CH_2)_2OCH_3$, et les substituants $R_1$ sont identiques ou différents.

**5.** Composé pour son utilisation selon l'une quelconque des revendications 1 à 3, dans lequel quand n vaut 2, alors les deux $R_1$ forment avec le groupe phényl aux positions méta et para un groupe dioxolane ou un groupe 1,4 dioxane.

**6.** Composé pour son utilisation selon l'une quelconque des revendications 1, 2 et 4, dans lequel $R_3$ est H et $R_4$ est un phényle non-substitué ou substitué par un ou deux groupes identiques ou différents et qui est/sont sélectionné parmi OH, $OCH_3$ et Halo, de préférence chloro.

**7.** Composé de formule générale (I) :

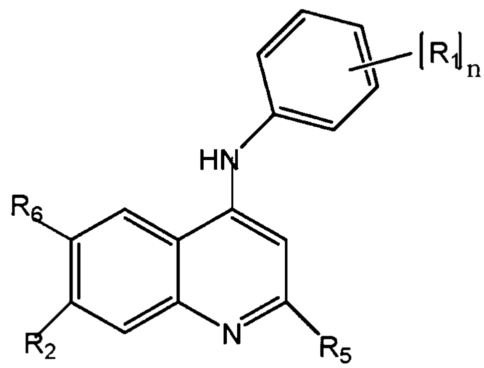

(I)

dans laquelle :

$R_1$ est sélectionné parmi alkyle, Halo, OH, O-alkyle, $NH_2$, NH-alkyle, N-(alkyle)$_2$, S-alkyle, $CF_3$, $OCF_3$, $OCF_2H$ et $O(CH_2)_{n1}O(CH_2)_{n2}CH_3$, $n_1$ valant de 1 à 4 et $n_2$ valant de 0 à 3, Halo étant sélectionné parmi Cl, F, Br et I;
n vaut 1 ou 2,
quand n vaut 2, alors il est aussi possible pour $R_1$ de former avec le groupe phényle aux positions méta et para un groupe dioxolane ou un groupe 1,4 dioxane,
$R_2$ est sélectionné parmi H et $NR_3R_4$,
$R_3$ est H et $R_4$ est un phényl optionnellement substitué par un ou plusieurs substituants sélectionnés parmi OH, O-alkyle et Halo,
ou $R_3$ et $R_4$ forment ensemble une chaine $(CH_2)_2O(CH_2)_2$, c'est-à-dire que $NR_3R_4$ forme un groupe morpholino,
$R_5$ est sélectionné parmi H, radical cyclique, O-alkyle, O-$(CH_2)_{n1}$-(radical cyclique), et $(CH_2)_{n1}$-(radical cyclique), $n_1$ étant défini comme précédemment, ledit radical cyclique étant sélectionné parmi

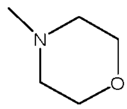 et 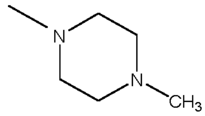,

$R_6$ est sélectionné parmi H et Halo,
ou un sel pharmaceutiquement acceptable et/ou un isomère optique, un tautomère ou un solvate de celui-ci, pour son utilisation dans le traitement d'une maladie sélectionnée parmi les lymphomes et la leucémie.

**8.** Composé pour son utilisation selon la revendication 1, dans lequel ledit composé est sélectionné parmi :

- *N*-(3,5-difluorophényl)-7-chloroquinoléin-4-amine,
- *N-(*4-hydroxy-3-chlorophényl)-7-chloroquinoléin-4-amine,
- *N*-(3,5-difluorophényl)-7-morpholinoquinoléin-4-amine,
- *N*-(4-(difluorométhoxy)phényl)-7-chloro-quinoléin-4-amine,
- *N*-(4-méthoxy-3-(2-méthoxyéthoxy)phényl)-7-chloro-quinoléin-4-amine,
- *N*-(2,4-diméthoxyphényl)-6-chloroquinoléin-4-amine,
- *N*-(3,4-diméthoxyphényl)-7-chloroquinoléin-4-amine,
- *N*-(2,4-diméthoxyphényl)-7-chloroquinoléin-4-amine,
- *N4*-(3,5-difluorophényl)-*N7*-(4-méthoxyphényl)quinoléin-4,7-diamine,
- *N4*-(3,5-difluorophényl)-*N7*-(3-chloro-4-hydroxyphényl)quinoléin-4,7-diamine,
- *N4*-(3,5-difluorophényl)-*N7*-(3-méthoxyphényl)quinoléin-4,7-diamine,
- *N*-(3,4-diméthoxyphényl)-7-chloro-2-morpholino-quinoléin-4-amine,
- *N*-(3,4-diméthoxyphényl)-7-chloro-2-(4-méthylpipérazin-1-yl)-quinoléin-4-amine,
- *N*-(3,4-diméthoxyphényl)-7-chloro-2-(2-morpholinoéthoxy)quinoléin-4-amine,
- *N*-(3,4-diméthoxyphényl)-7-chloro-2-méthoxy-quinoléin-4-amine,
- 7-chloro-*N*-(2,3-dihydroxybenzo[b][1,4]dioxin-6-yl)quinoléin-4-amine, et
- 7-chloro-*N*-(benzo[d][1,3]dioxol-5-yl)quinoléin-4-amine.

**9.** Composé pour son utilisation selon l'une quelconque des revendications 1, 2, 4 et 6, dans lequel ledit composé est sélectionné parmi :

- $N^4$-(3,5-difluorophényl)- $N^7$- (4-méthoxyphényl)quinoléin-4,7-diamine,
- $N^4$-(3,5-difluorophényl)-$N^7$-(3-chloro-4-hydroxyphényl)quinoléin-4,7-diamine, et
- $N^4$-(3,5-difluorophényl)-$N^7$-(3-méthoxyphényl)quinoléin-4,7-diamine.

**10.** Composé pour son utilisation selon l'une quelconque des revendications 1, 3 et 4, dans lequel ledit composé est sélectionné parmi :

- *N*-(3,4-diméthoxyphényl)-7-chloro-2-morpholino-quinoléin-4-amine,
- *N*-(3,4-diméthoxyphényl)-7-chloro-2-(4-méthylpipérazin-1-yl)-quinoléin-4-amine,
- *N*-(3,4-diméthoxyphényl)-7-chloro-2-(2-morpholinoéthoxy)quinoléin-4-amine et
- *N*-(3,4-diméthoxyphényl)-7-chloro-2-méthoxy-quinoléin-4-amine.

**11.** Composé pour son utilisation selon la revendication 1, dans lequel ledit composé est sélectionné parmi :

- 7-chloro-N-(4-(2-méthoxyéthoxy)phényl)quinoléin-4-amine et
- 7-chloro-N-(3-(hydroxy)-4-(méthoxy)phényl)quinoléin-4-amine.

**12.** Composé pour son utilisation selon la revendication 7, dans lequel ledit composé est sélectionné parmi :

- *N*-(4-trifluorométhoxyphényl)-6-chloroquinoléin-4-amine,
- *N*-(p-tolyl)-7-morpholinoquinoléin-4-amine,
- *N-(*o-tolyl)-7-morpholinoquinoléin-4-amine,
- *N-(*4-méthoxyphényl)-7-morpholinoquinoléin-4-amine,
- *N*-(3-méthoxyphényl)-7-morpholinoquinoléin-4-amine,
- *N*-(3-trifluorométhyl)phényl)-7-morpholinoquinoléin-4-amine,
- *N4,N7*-bis(4-methoxyphényl)quinoléin-4,7-diamine,

- *N4,N7*-bis(3-méthoxyphényl)quinoléin-4,7-diamine,
- *N4*-(p-tolyl)-N7-(3-méthoxyphényl)quinoléin-4,7-diamine,
- *N4*-(p-tolyl)-N7-(4-méthoxyphényl)quinoléin-4,7-diamine,
- *N4*-(3-(trifluorométhyl)phényl)-N7-(4-méthoxyphényl)quinoléin-4,7-diamine,
- *N4-(*o-tolyl)-N7-(3-méthoxyphényl)quinoléin-4,7-diamine,
- *N4-(*4-méthoxyphényl)-N7-(3-méthoxyphényl)quinoléin-4,7-diamine, et
- *N4*-(3-méthoxyphényl)-N7-(4-méthoxyphényl)quinoléin-4,7-diamine.

**13.** Composition pharmaceutique pour son utilisation dans le traitement d'une maladie sélectionnée parmi les lymphomes et la leucémie comprenant :

- un composé tel que défini dans l'une quelconque des revendications 1 à 12,
- optionnellement, au moins un véhicule pharmaceutiquement acceptable.

**14.** Composition pharmaceutique pour son utilisation selon la revendication 13, comprenant additionnellement :

- au moins un composé immunomodulateur, ledit composé immunomodulateur étant de préférence un anticorps immunomodulateur, et plus préférablement encore un anticorps choisi parmi un anticorps anti-PD1, un anticorps anti-CTLA4, un anticorps anti-PD-L1 et un mélange de deux ou plusieurs de ceux-ci, et/ou
- au moins un autre agent thérapeutique.

**15.** Composition pharmaceutique pour son utilisation selon la revendication 14, en tant que préparation combinée pour une utilisation simultanée, séparée ou séquentielle.

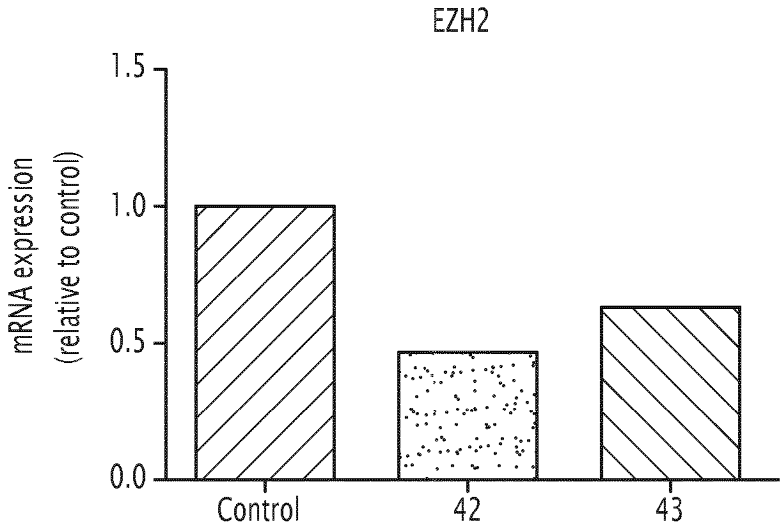


FIG.1

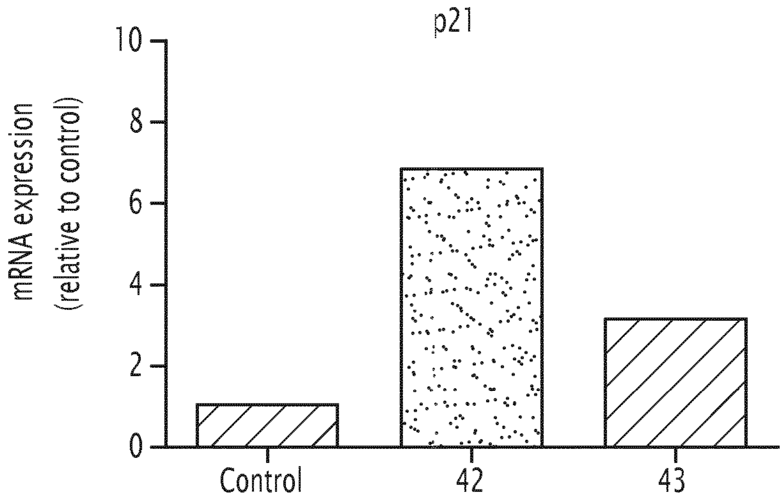


FIG.2

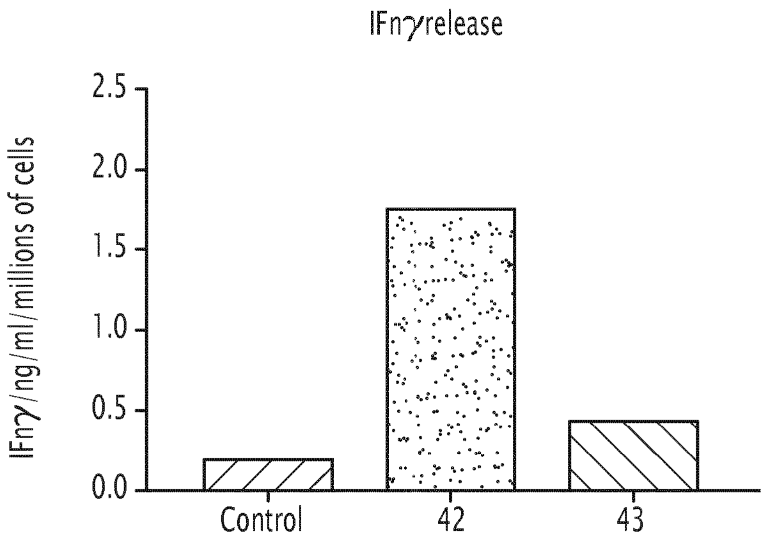


FIG.3

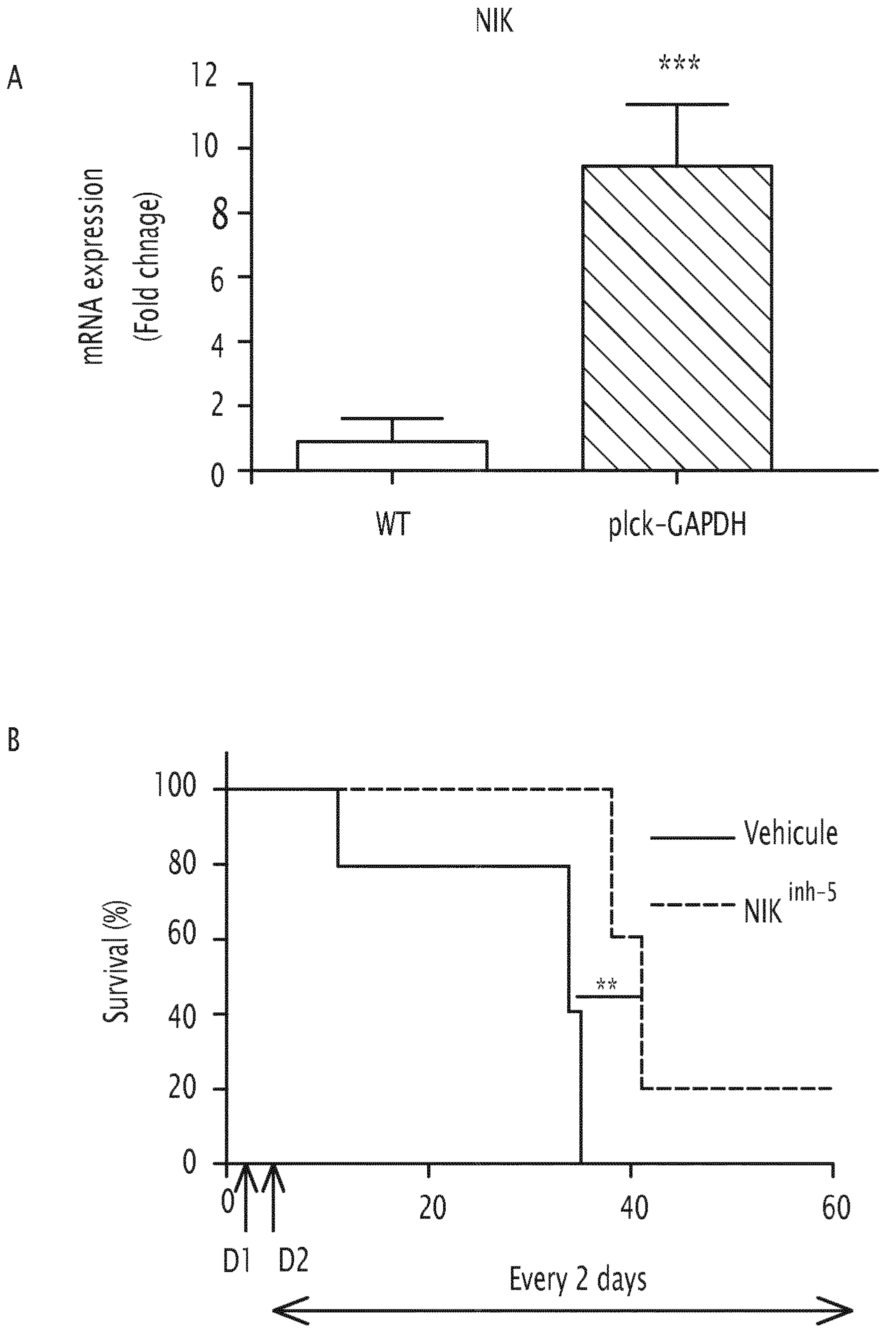
NIK
A
mRNA expression
(Fold chnage)
12
10
8
6
4
2
0
***
WT
plck-GAPDH
B
Survival (%)
100
80
60
40
20
0
Vehicule
NIK inh-5
**
0
20
40
60
D1
D2
Every 2 days

FIG.4

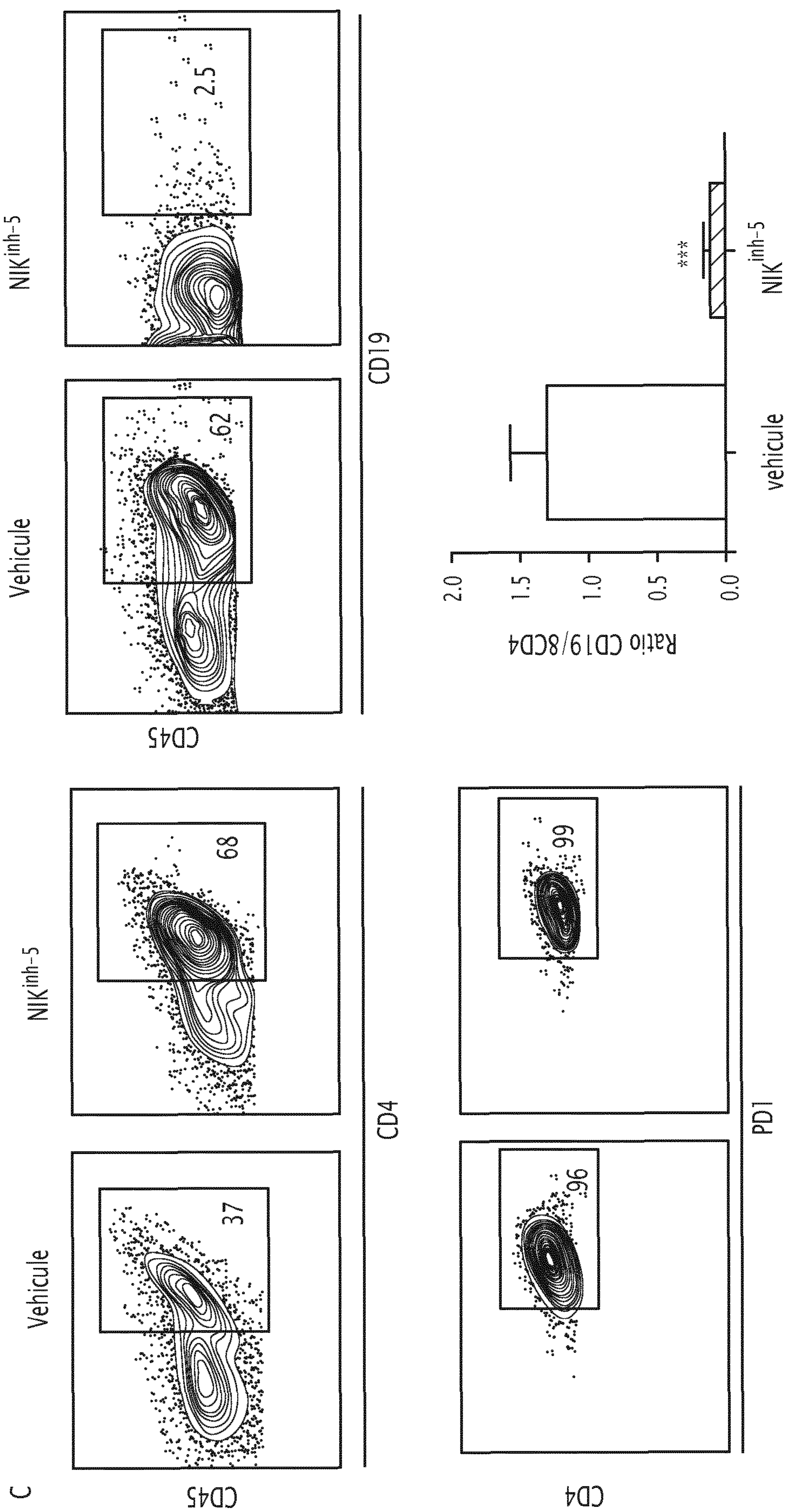
C
Vehicule
NIK$^{inh-5}$
CD45
37
68
CD4
96
99
PD1
62
2.5
CD19
Ratio CD19/8CD4
0.0
0.5
1.0
1.5
2.0
***
vehicule

FIG.4

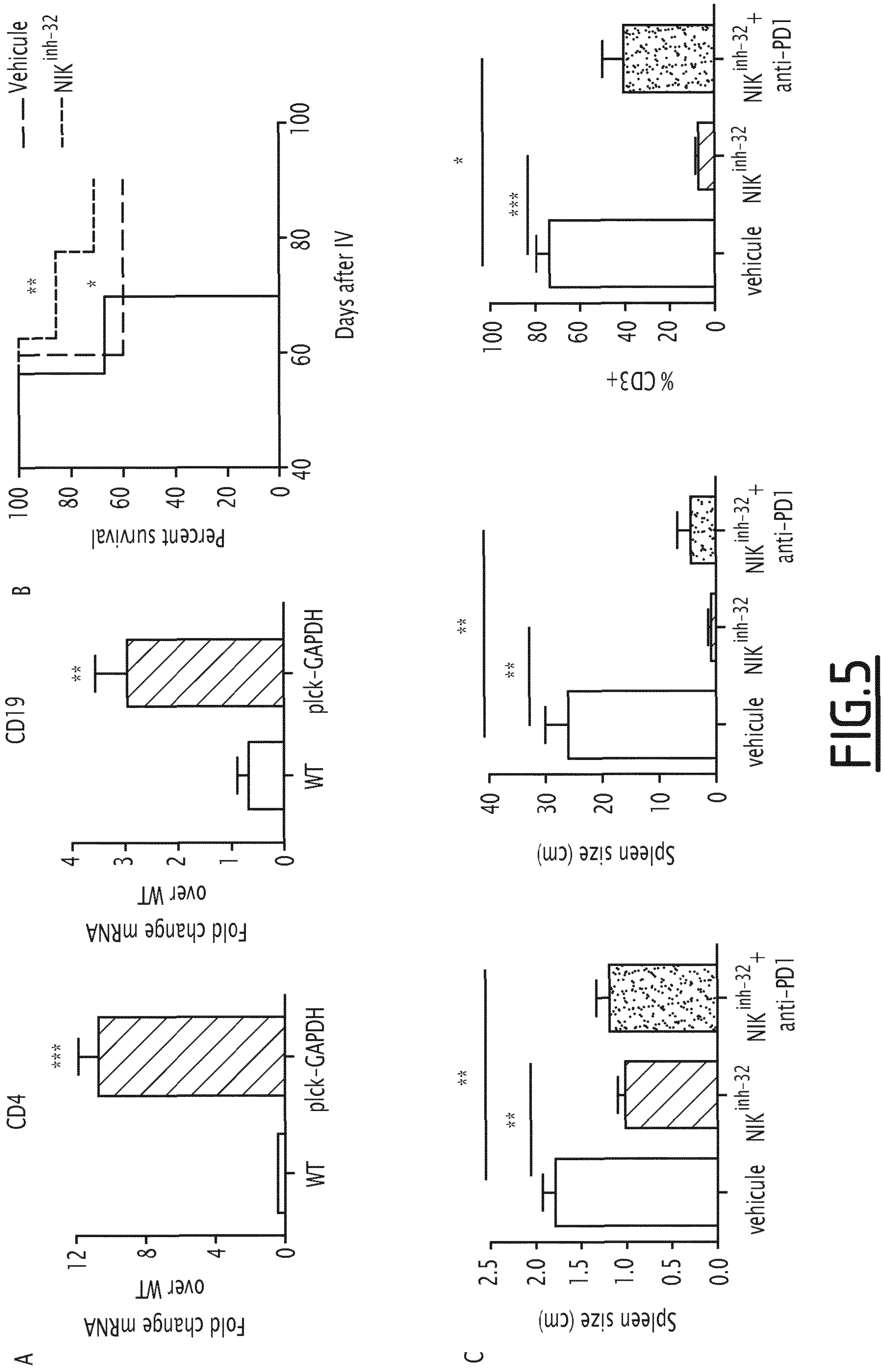
A
CD4
Fold change mRNA over WT
WT
plck-GAPDH
***
CD19
**
B
Percent survival
Days after IV
Vehicule
NIK inh-32
*
**
C
Spleen size (cm)
vehicule
NIK inh-32
NIK inh-32 + anti-PD1
% CD3+
***

FIG.5

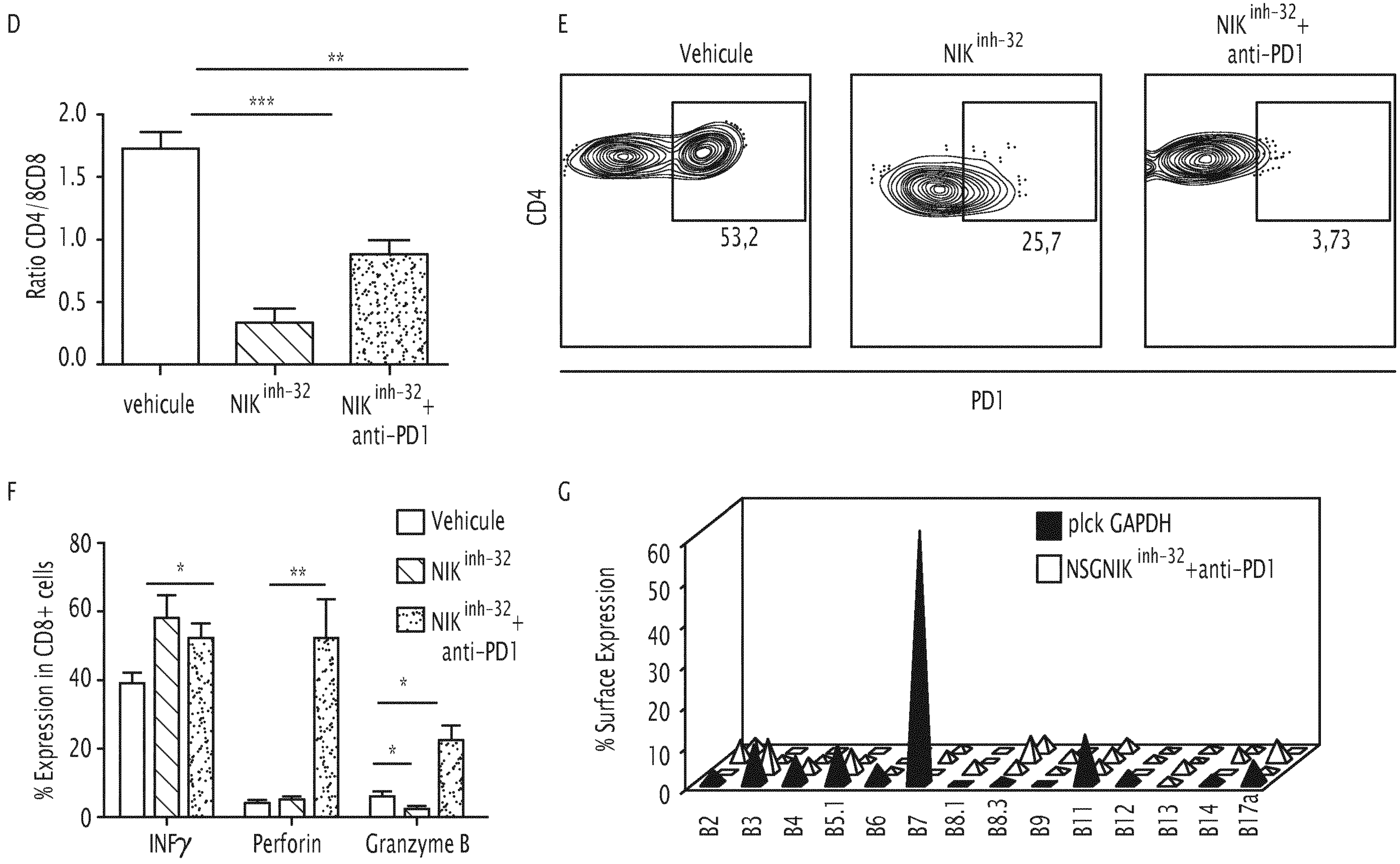


FIG.5CONTINUED

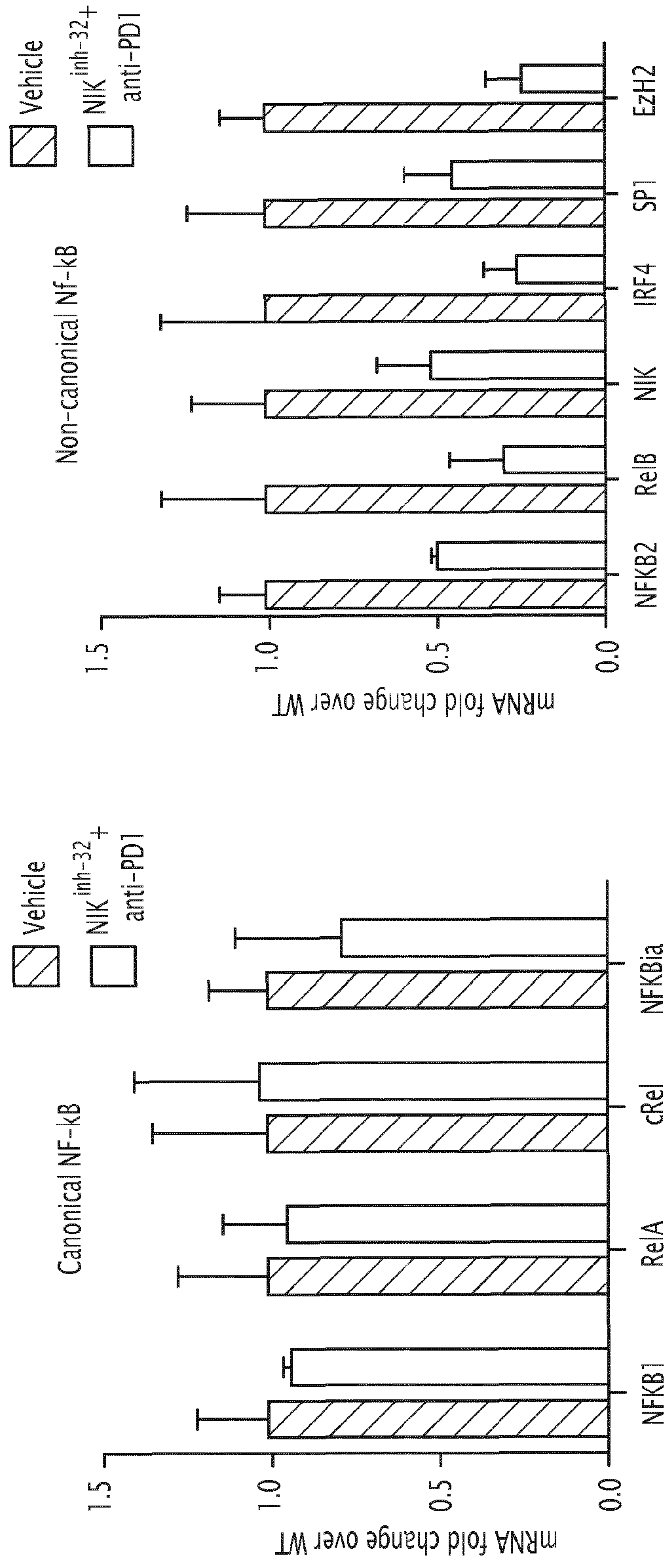

Canonical NF-kB
Vehicle
NIK inh-32 + anti-PD1
mRNA fold change over WT
1.5
1.0
0.5
0.0
NFKB1
RelA
cRel
NFKBia
Non-canonical Nf-kB
Vehicle
NIK inh-32 + anti-PD1
mRNA fold change over WT
1.5
1.0
0.5
0.0
NFKB2
RelB
NIK
IRF4
SP1
EzH2


FIG.5END

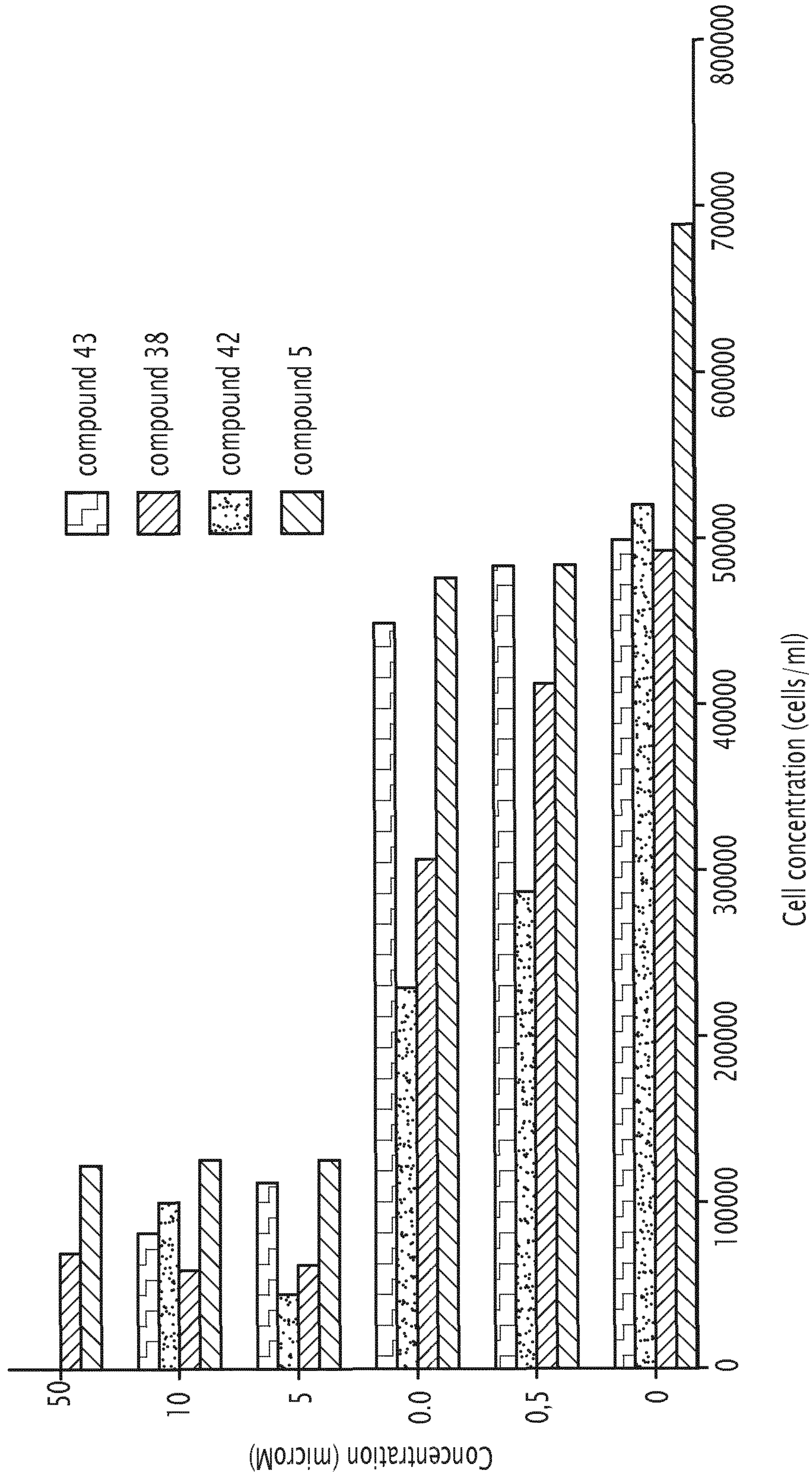
compound 43
compound 38
compound 42
compound 5
Concentration (microM)
50
10
5
0.0
0,5
0
Cell concentration (cells/ml)
0
100000
200000
300000
400000
500000
600000
700000
800000

FIG.6

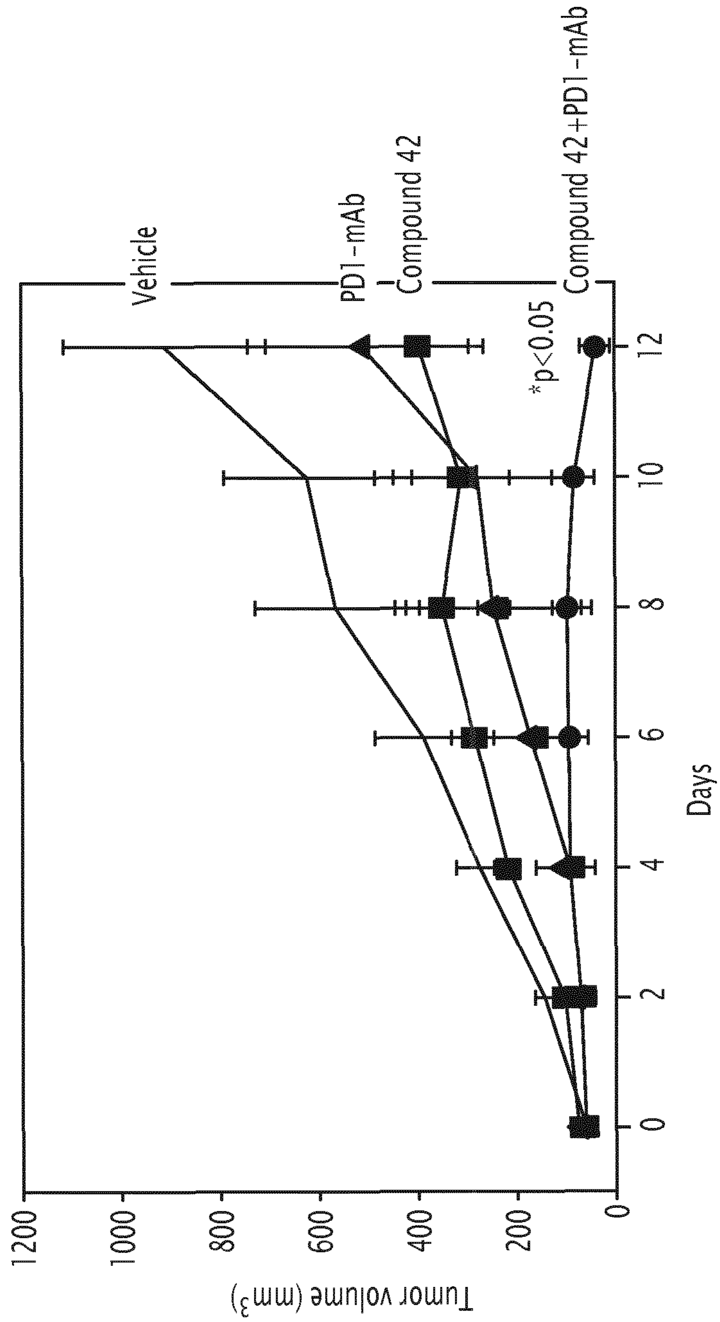
Vehicle
PD1-mAb
Compound 42
Compound 42+PD1-mAb
*p<0.05
Tumor volume (mm³)
1200
1000
800
600
400
200
0
0
2
4
6
8
10
12
Days

FIG.7

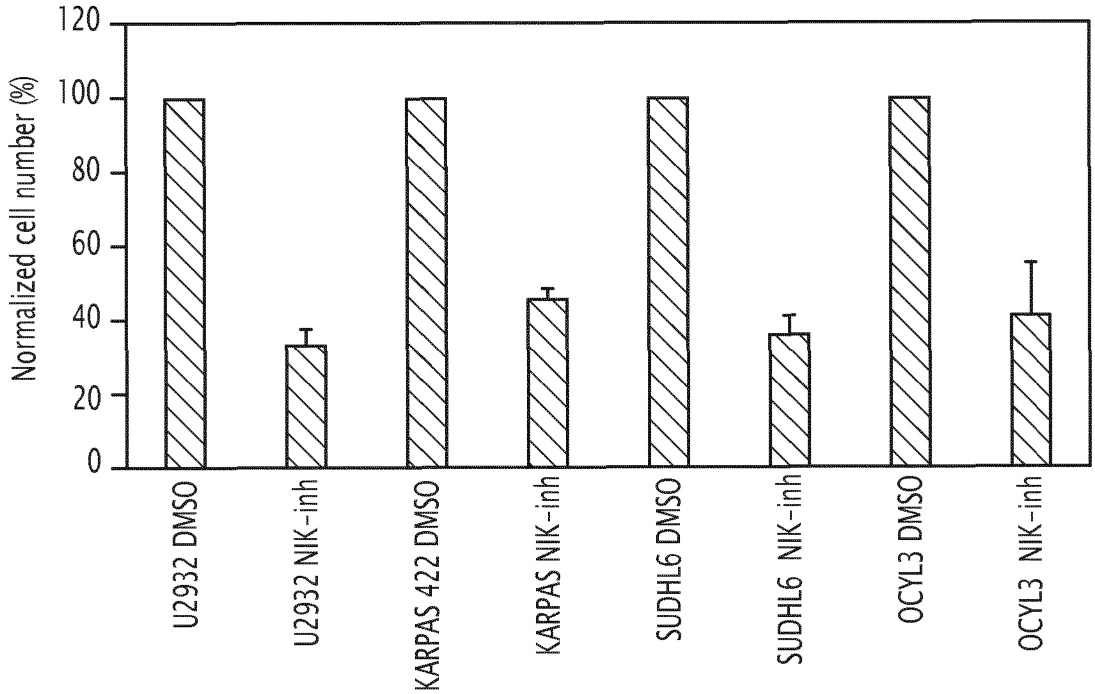
120
100
80
60
40
20
0
Normalized cell number (%)
U2932 DMSO
U2932 NIK-inh
KARPAS 422 DMSO
KARPAS NIK-inh
SUDHL6 DMSO
SUDHL6 NIK-inh
OCYL3 DMSO
OCYL3 NIK-inh

FIG.8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 102846623
- US 3538214 A
- US 4060598 A
- US 4173626 A
- US 3119742 A
- US 3492397 A
- WO 2018007648 A
- EP 2017067306 W


### Non-patent literature cited in the description


- Remington's Pharmaceutical Sciences. Mack Publishing Company, 1995
- *Bioorg. Med. Chem.*, 2013, vol. 21 (11), 3147-3153
- *J. Med. Chem.*, 2015, vol. 58 (14), 5522-5537
- *Journal of the American Chemical Society*, 1946, vol. 68, 1807-1808
- *Angewandte Chemie, International edition*, 1995, vol. 34, 1348-1350
- *Tetrahedron Letters*, 2013, vol. 54, 6900-6904
- **STAHL** ; **WERMUTH**. Handbook of Pharmaceutical Salts: Properties, Selection, and Use. Wiley-VCH, 2002
- **HALEBLIAN**. *J Pharm Sci*, August 1975, vol. 64 (8), 1269-1288
- **E. L. ELIEL**. Stereochemistry of Organic Compounds. Wiley, 1994
- **G. SUBRAMANIAN**. Chiral Separation Techniques. John Wiley & Sons, 2008
- **G. B. COX**. Preparative Enantioselective Chromatography. Wiley, 2005
- **J. FU** ; **I. J. MALM** ; **D. K. KADAYAKKARA** ; **H. LEVITSKY** ; **D. PARDOLL** ; **Y. J. KIM**. Preclinical evidence that PD1 blockade cooperates with cancer vaccine TEGVAX to elicit regression of established tumors.. *Cancer research*, 2014, vol. 74, 4042-4052
- **T. BALD** ; **J. LANDSBERG** ; **D. LOPEZ-RAMOS** ; **M. RENN** ; **N. GLODDE** ; **P. JANSEN** ; **E. GAFFAL** ; **J. STEITZ** ; **R. TOLBA** ; **U. KALINKE**. Immune cell-poor melanomas benefit from PD-1 blockade after targeted type I IFN activation. *Cancer discovery*, 2014, vol. 4, 674-687
- **Z. GUO** ; **H. WANG** ; **F. MENG** ; **J. LI** ; **S. ZHANG**. Combined Trabectedin and anti-PD1 antibody produces a synergistic antitumor effect in a murine model of ovarian cancer. *J Transl Med*, 2015, vol. 13, 247
- **H. E. TEULINGS** ; **J. LIMPENS** ; **S. N. JANSEN** ; **A. H. ZWINDERMAN** ; **J. B. REITSMA** ; **P. I. SPULS** ; **R. M. LUITEN**. Vitiligo-like depigmentation in patients with stage III-IV melanoma receiving immunotherapy and its association with survival: a systematic review and meta-analysis.. *J Clin Oncol*, 2015, vol. 33, 773-781
- **H. E. TEULINGS** ; **M. OVERKAMP** ; **E. CEYLAN** ; **L. NIEUWEBOER-KROBOTOVA** ; **J. D. BOS** ; **T. NIJSTEN** ; **A. W. WOLKERSTORFER** ; **R. M. LUITEN** ; **J. P. VAN DER VEEN**. Decreased risk of melanoma and nonmelanoma skin cancer in patients with vitiligo: a survey among 1307 patients and their partners.. *Br J Dermatol*, 2013, vol. 168, 162-171
- **M. RASHIGHI** ; **P. AGARWAL** ; **J. M. RICHMOND** ; **T. H. HARRIS** ; **K. DRESSER** ; **M. W. SU** ; **Y. ZHOU** ; **A. DENG** ; **C. A. HUNTER** ; **A. D. LUSTER**. CXCL10 is critical for the progression and maintenance of depigmentation in a mouse model of vitiligo. *Sci Transl Med*, 2014, vol. 6, 223-223
- **G. M. DE DONATIS** ; **E. L. PAPE** ; **A. PIERRON** ; **Y. CHELI** ; **V. HOFMAN** ; **P. HOFMAN** ; **M. ALLEGRA** ; **K. ZAHAF** ; **P. BAHADORAN** ; **S. ROCCHI**. NF-kB2 induces senescence bypass in melanoma via a direct transcriptional activation of EZH2. *Oncogene*, 2015
- **T. W. KANG** ; **T. YEVSA** ; **N. WOLLER** ; **L. HOENICKE** ; **T. WUESTEFELD** ; **D. DAUCH** ; **A. HOHMEYER** ; **M. GEREKE** ; **R. RUDALSKA** ; **A. POTAPOVA**. Senescence surveillance of pre-malignant hepatocytes limits liver cancer development.. *Nature*, 2011, vol. 479, 547-551
- **DE DONATIS GM** ; **LE PAPE E** ; **PIERRON A** ; **CHELI Y** ; **HOFMAN V** ; **HOFMAN P** ; **ALLEGRA M** ; **ZAHAF K** ; **BAHADORAN P** ; **ROCCHI S**. NF-kB2 induces senescence bypass in melanoma via a direct transcriptional activation of EZH2. *Oncogene*, May 2016, vol. 35 (21), 2813

- **AHSANUDDIN, A. N ; BRYNES, R. K ; LI, S.** Peripheral blood polyclonal plasmacytosis mimicking plasma cell leukemia in patients with angioimmunoblastic T-cell lymphoma: report of 3 cases and review of the literature.. *Int J Clin Exp Pathol*, 2011, vol. 4, 416-420
- **ALGUL, H. ; TREIBER, M ; LESINA, M ; NAKHAI, H ; SAUR, D ; GEISLER, F ; PFEIFER, A ; PAXIAN, S ; SCHMID, R. M.** Pancreas-specific RelA/p65 truncation increases susceptibility of acini to inflammation-associated cell death following cerulein pancreatitis.. *J Clin Invest*, 2007, vol. 117, 1490-1501
- **BACHMANN, I. M. ; HALVORSEN, O. J ; COLLETT, K ; STEFANSSON, I. M ; STRAUME, O ; HAUKAAS, S. A. ; SALVESEN, H. B ; OTTE, A. P ; AKSLEN, L. A.** EZH2 expression is associated with high proliferation rate and aggressive tumor subgroups in cutaneous melanoma and cancers of the endometrium, prostate, and breast. *J Clin Oncol*, 2006, vol. 24, 248-273
- **BERG, T. ; THOENE, S ; YAP, D ; WEE, T ; SCHOELER, N ; ROSTEN, P ; LIM, E ; BILENKY, M ; MUNGALL, A. J ; OELLERICH, T et al.** A transgenic mouse model demonstrating the oncogenic role of mutations in the polycomb-group gene EZH2 in lymphomagenesis. *Blood*, 2014, vol. 123, 3914-3924
- **CHEN, Z ; YANG, P ; LI, W. ; HE, F. ; WEI, J ; ZHANG, T ; ZHONG, J ; CHEN, H ; CAO, J.** Expression of EZH2 is associated with poor outcome in colorectal cancer. *Oncol Lett*, 2018, vol. 15, 2953-2961
- **CHICHE, J. ; POMMIER, S ; BENETEAU, M ; MONDRAGON, L ; MEYNET, O ; ZUNINO, B ; MOUCHOTTE, A ; VERHOEYEN, E ; GUYOT, M ; PAGES, G et al.** GAPDH enhances the aggressiveness and the vascularization of non-Hodgkin's B lymphomas via NF-kappaB-dependent induction of HIF-1alpha.. *Leukemia*, 2015, vol. 29, 1163-1176
- **CILDIR, G ; LOW, K. C ; TERGAONKAR, V.** Noncanonical NF-kappaB Signaling in Health and Disease.. *Trends Mol Med*, 2016, vol. 22, 414-429
- **COLELL, A. ; GREEN, D. R ; RICCI, J. E.** Novel roles for GAPDH in cell death and carcinogenesis. *Cell Death Differ*, 2009, vol. 16, 1573-1581
- **DE LEVAL, L. ; GISSELBRECHT, C. ; GAULARD, P.** Advances in the understanding and management of angioimmunoblastic T-cell lymphoma.. *Br J Haematol*, 2010, vol. 148, 673-689
- **GAULARD, P ; DE LEVAL, L.** The microenvironment in T-cell lymphomas: emerging themes.. *Semin Cancer Biol*, 2014, vol. 24, 49-60
- **JACQUIN, M. A ; CHICHE, J ; ZUNINO, B. ; BENETEAU, M ; MEYNET, O ; PRADELLI, L. A ; MARCHETTI, S ; CORNILLE, A ; CARLES, M ; RICCI, J. E.** GAPDH binds to active Akt, leading to Bcl-xL increase and escape from caspase-independent cell death. *Cell Death Differ*, 2013, vol. 20, 1043-1054
- **LEMONNIER, F. ; MAK, T. W.** Angioimmunoblastic T-cell lymphoma: more than a disease of T follicular helper cells. *J Pathol*, 2017, vol. 242, 387-390
- **MORIN, R. D. ; JOHNSON, N. A ; SEVERSON, T. M ; MUNGALL, A. J ; AN, J ; GOYA, R ; PAUL, J. E ; BOYLE, M ; WOOLCOCK, B. W ; KUCHENBAUER, F et al.** Somatic mutations altering EZH2 (Tyr641) in follicular and diffuse large B-cell lymphomas of germinal-center origin.. *Nat Genet*, 2010, vol. 42, 181-185
- **NAGOSHI, H ; KURODA, J ; KOBAYASHI, T ; MAEGAWA, S ; CHINEN, Y ; KIYOTA, M ; NAKAYAMA, R ; MIZUTANI, S ; SHIMURA, Y ; YAMAMOTO-SUGITANI, M et al.** Clinical manifestation of angioimmunoblastic T-cell lymphoma with exuberant plasmacytosis. *Int J Hematol*, 2013, vol. 98, 366-374
- **ODQVIST, L. ; SANCHEZ-BEATO, M ; MONTES-MORENO, S ; MARTIN-SANCHEZ, E ; PAJARES, R ; SANCHEZ-VERDE, L. ; ORTIZ-ROMERO, P. L ; RODRIGUEZ, J ; RODRIGUEZ-PINILLA, S. M ; INIESTA-MARTINEZ, F et al.** NIK controls classical and alternative NF-kappaB activation and is necessary for the survival of human T-cell lymphoma cells.. *Clin Cancer Res*, 2013, vol. 19, 2319-2330
- **PARDOLL, D. M.** The blockade of immune checkpoints in cancer immunotherapy.. *Nat Rev Cancer*, 2012, vol. 12, 252-264
- **POSTOW, M. A. ; CALLAHAN, M. K. ; WOLCHOK, J. D.** Immune Checkpoint Blockade in Cancer Therapy.. *J Clin Oncol*, 2015, vol. 33, 1974-1982
- **SUN, S. C.** The non-canonical NF-kappaB pathway in immunity and inflammation. *Nat Rev Immunol*, 2017, vol. 17, 545-558
- **TOPALIAN, S. L. ; DRAKE, C. G ; PARDOLL, D. M.** Targeting the PD-1/B7-H1(PD-L1) pathway to activate anti-tumor immunity.. *Curr Opin Immunol*, 2012, vol. 24, 207-212